# EUROPEAN PATENT APPLICATION

(11) **EP 3 699 301 A1**
(43) Date of publication of application: **26.08.2020**
(21) Application number: 19460010.2
(22) Date of filing: 21.02.2019
(51) Int. Cl.: C12Q 1/6886

(54) **NOVEL RECQL4 MUTEINS AND PERSONALIZED TREATMENT OF GLIOBLASTOMA PATIENTS**

(71) Applicant: Ryvu Therapeutics S.A., 30-348 Krakow (PL)
(72) Inventor: Kaminska-Kaczmarek, Bozena, 02-093 Warsaw (PL); Krol, Sylwia Katarzyna, 01-234 Warsaw (PL); Kaczmarczyk, Agnieszka, 02-093 Warsaw (PL); Wojtas, Bartosz, 02-093 Warsaw (PL)
(74) Representative: Ledl, Andreas

(57) **Abstract**

The present invention provides novel muteins of human ATP-dependent DNA helicase Q4 (RecQL4) and polynucleotides encoding them. Further, the present invention relates to *in vitro* methods of determining the susceptibility of glioblastoma patients to therapeutic agents, as well as to the personalized treatment of the identified glioblastoma patients.

## Description

### Technical field

The present invention relates to muteins of human ATP-dependent DNA helicase Q4 (RecQL4) and polynucleotides encoding them. Further, the present invention relates to *in vitro* methods of determining the susceptibility of glioblastoma patients to therapeutic agents, as well as to the personalized treatment of the identified glioblastoma patients.

### Background of the Invention

Gliomas originate from neural or progenitor stem cells and represent over 70% of all brain malignancies. Based on histopathological characteristics, the World Health Organization (WHO) divided gliomas into the following groups: low-grade gliomas (LGG, grades I and II) are well-differentiated, slow-growing tumors, whereas high-grade gliomas (HGG, grades III and IV) are less differentiated or anaplastic, and strongly infiltrate brain parenchyma. The most common and deadliest primary brain tumor is glioblastoma (GBM, grade IV), an aggressive, highly diffusive and vascularized astrocytic tumor. GBM is one of the most difficult human malignancies to treat due to frequent dysfunction of tumor suppressors and oncogenes. Despite advances in surgery, radiotherapy and chemotherapy current therapies against malignant gliomas are not effective (Stupp et al., N. Engl. J. Med. 352 (2005), 987-996).

Glioblastoma development is a multistep process, involving genetic and epigenetic alterations. The exome sequencing of GBMs uncovered mutations in many genes involved in epigenetic regulation, including genes coding for histone deacetylases HDAC2 and HDAC9, histone methyltransferases SETDZ, SET7, SETD7, MLL3, MLL4, methyl-CpG binding domain protein 1 and chromatin remodelers SMARCAZ, SMARCA4 (Maleszewska and Kaminska, Future Oncol. 11 (2015), 2587-2601).

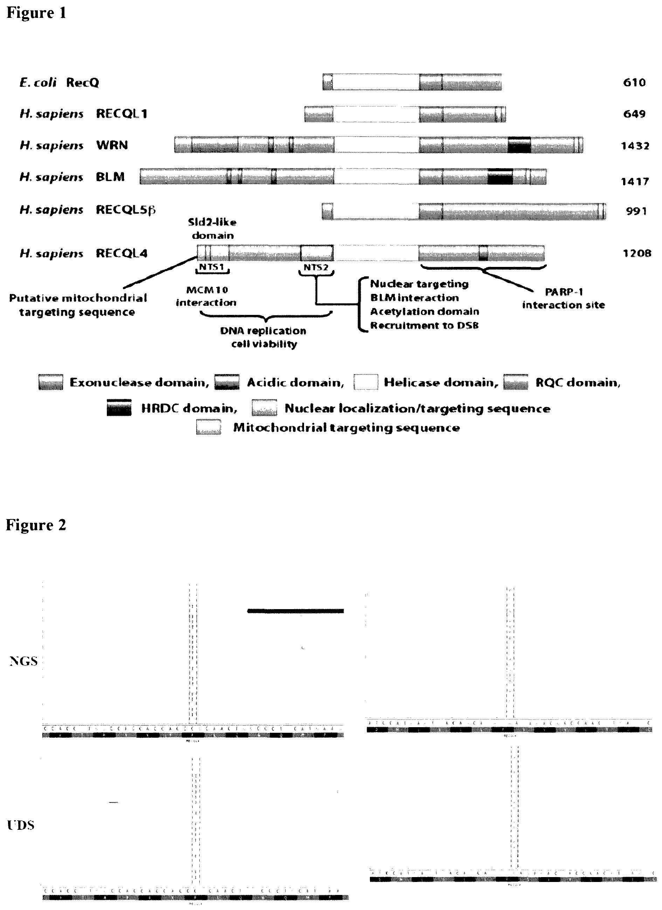

DNA helicases of the RecQ family are ATP-dependent enzymes, highly conserved between bacteria, yeasts, and all higher eukaryotes. These helicases participate in many aspects of DNA metabolism: replication, recombination, transcription, DNA repair and telomere maintenance. RecQ helicases participate in all phases of DNA replication (including initiation, Okazaki fragment processing, leading- and lagging-strand elongation, and resolution) and other replication associated events. Therefore, they are often referred to as "guardians of the genome". RecQ helicases demonstrate specific and preferred binding to DNA substrates that resemble DNA repair intermediates. They also interact physically with each other, and their roles in different DNA metabolic pathways may be both overlapping and complementary, creating a functional complexity (Fig.1). In human cells, there are five RecQ family members: RECQL1, BLM, WRN, RECQL4, and RECQL5 (Croteau et al., Ann. Rev. Biochem. 83 (2014), 519-552). RECQL4 belongs to the RecQ family of DNA helicases, enzymes important for maintaining genomic integrity and stability.

Wibom et al. (Acta Oncologica 51 (2012), 325-332) describe that DNA-repair gene variants are associated with glioblastoma survival. It was found previously that nine SNPs annotating MSH12, RAD51L1 and RECQL4 were associated with glioblastoma survival. Of all the analysed SNPs within the RECQL4 gene, one was non-synonymous. However, this SNP did not turn out to be significantly associated with survival in the study, whereas two polymorphisms in introns showed a significant association with survival.

Temozolomide (TMZ) has been used as a standard therapy for the treatment of newly diagnosed glioblastoma multiforme since 2005. A meta-analysis showed that the overall clinical benefit rate was 50.5% and the overall 6-month PFS rate was found to be 27.8% (Chen et al. Eur J Neurol. 2013 Feb;20(2):223-30).

In view thereof, there is a need for the provision of means and methods for the identification of glioblastoma patients being susceptible to therapy as well as means and methods for the personalized treatment of the identified patients.

### Summary of the Invention

The technical problems underlying the invention are solved by the provision of the subject-matter as defined in the claims.

According to a first aspect, an ATP-dependent DNA helicase Q4 (RecQL4) mutein is provided, wherein the RecQL4 mutein differs from the wild-type sequence (SEQ ID NO:1) by one or more substitutions selected from P532S and R766Q of the wild-type human RecQL4 amino acid sequence of SEQ ID NO:1.

According to a second aspect, a polynucleotide encoding the mutein of the first aspect is provided.

In an embodiment of the second aspect, the nucleotide sequence of the polynucleotide sequence differs from the wild-type nucleotide sequence (SEQ ID NO:4) by one or more substitutions selected from the codon substitution CCC/TCC encoding the amino acid at position 532 of SEQ ID NO:1 and the codon substitution CGG/CAG encoding the amino acid at position 766 of SEQ ID NO: 1.

In an embodiment of the second aspect, the polynucleotide comprises the nucleotide sequence of SEQ ID NO:5.

In an embodiment of the second aspect, the polynucleotide comprises the nucleotide sequence of SEQ ID NO:6.

According to a third aspect, there is provided an *in vitro* method of determining the susceptibility for imidazotetrazine drug treatment of a human glioblastoma patient comprising
a) providing a nucleic acid sample from the human glioblastoma patient;
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of one or more of said mutations is indicative for the susceptibility of the human glioblastoma patient for imidazotetrazine drug treatment.

According to a fourth aspect, there is provided an *in vitro* method of determining the susceptibility for PARP inhibitor treatment of a human glioblastoma patient comprising
a) providing a nucleic acid sample from the human glioblastoma patient;
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of one or more of said mutations is indicative for the susceptibility of the human glioblastoma patient for PARP inhibitor treatment.

According to a fifth aspect, an imidazotetrazine drug for use in the treatment of glioblastoma in a human patient is provided, wherein the patient has been identified in the method according to the third aspect of the invention.

According to a sixth aspect, a PARP inhibitor for use in the treatment of glioblastoma in a human patient is provided, wherein the human patient has been identified in the method according to the fourth aspect of the invention.

According to a seventh aspect, a kit is provided for the analysis of a single nucleotide polymorphism indicative for the susceptibility for imidazotetrazine drug or PARP inhibitor treatment of a human glioblastoma patient, comprising at least one primer and/or probe for determining the genotype at rs763278718.

According to an eighth aspect, a kit is provided for the analysis of a single nucleotide polymorphism indicative for the susceptibility for imidazotetrazine drug or PARP inhibitor treatment of a human glioblastoma patient, comprising at least one primer and/or probe for determining the genotype at rs931761657.

The inventors surprisingly found that in one out of 200 samples of WHO II, III, and IV grade glioma samples (derived from patients; 134 samples from WHO grade IV samples, i.e. patients suffering from glioblastoma), the codon CCC encoding the amino acid proline at position 532 of the wild-type RECQL4 amino acid sequence (SEQ ID NO:1) has been substituted by the codon TCC encoding the amino acid serine. It has further been observed in two samples out of the 200 samples of WHO II, III and IV grade glioma samples as mentioned above that the codon CGG encoding the amino acid arginine at position 766 of the wild-type amino acid sequence (SEQ ID NO:1) has been substituted by the codon CAG encoding the amino acid glutamine. The three samples discussed above were WHO grade IV-samples, i.e. derived from patients suffering from glioblastoma. Moreover, in the afore-mentioned cohort of 200 samples of WHO II, III and IV grade glioma samples, in total 12 samples with possibly damaging/deleterious variants in the RECQL4 gene were found. 10 of the 12 samples were from WHO grade IV samples, i.e. from patients suffering from glioblastoma.

Based on structural homology using the crystal structure from the RecQ homolog in *E. coli* it was concluded that P532 is located in a close proximity of the bound ssDNA and the catalytic site for ATP hydrolysis, which clearly indicates a functional relevance. In addition, a functional prediction server (PROVEAN) indicated that changing proline at position 532 (PS32) into any other amino acid would indeed lead to deleterious effects. Structural comparisons with human BLM helicases loaded with either ADP or ADP and DNA (Newman et al., Nucleic Acids Res. 43 (2015), 5221-5235) show that alterations in the position R766Q could also have some influence of protein function.

Functional studies using RECQL4 knock-down and knock-out glioma cells have been performed by the inventors, which are described in detail in the example section of the present application. It could be shown in the siRNA knock-down as well as in the CRISPR/Cas9 knockout-models that - whereas the temozolomide treatment of control glioma cells did not result in any inhibition of cell viability and/or proliferation - the temozolomide treatment of RecQL4 knock-down and RecQL4 knock-out glioma cells resulted in a significant inhibition of cell proliferation. Also for the PARP inhibitor olaparib, the results showed that - whereas olaparib treatment of control glioma cells did not result in any inhibition of cell viability and/or proliferation - olaparib treatment of RecQL4 knock-down and RecQL4 knock-out glioma cells significantly inhibits cell proliferation.

The structural modelling results together with the functional studies on the knock-down and knock-out, respectively, are very strong indicators and clearly support an approach of personalized treatment for glioblastoma, wherein the glioblastoma patients that have been identified according to the third and fourth aspect of the present invention are more susceptible to treatment than glioblastoma patients who do not carry at least one mutation at the identified positions in the RecQL4 protein involved in DNA repair.

### Brief Description of the Drawings

Figure 1 shows the location of functional domains in RECQL helicases.
Figure 2 shows a screenshot from Integrative Genome Browser (IGV) of a somatic variant in RECQL4 gene identified in GBM3210 (Glioblastoma multiforme) sample.
Figure 3 shows the position of the mutations (P532S, R766Q) in a preliminary structural homology modeling.
Figure 4 shows that RECQL4 is highly overexpressed in glioma cells.
Figure 5 relates to the effect of RecQL4 knockdown in glioma cell lines.
Figure 6 shows that siRNA knockdown of RECQL4 sensitizes glioma cells to temozolomide treatment.
Figure 7 shows that stable knock-out of RECQL4 sensitizes glioma cells to temozolomide treatment.
Figure 8 shows the effects of olaparib on viability (MTT) and proliferation (BrdU incorporation assay) on siRNA-treated glioma cells.
Figure 9 relates to the effects of olaparib on viability and proliferation on RECQL4 stable knock-out glioma cells.

### Detailed Description of the Invention

Where the term "comprise" or "comprising" is used in the present description and claims, it does not exclude other elements or steps. For the purpose of the present invention, the term "consisting of' is considered to be an optional embodiment of the term "comprising". If hereinafter a group is defined to comprise at least a certain number of embodiments, this is also to be understood to disclose a group which optionally consists only of these embodiments.

Where an indefinite or a definite article is used when referring to a singular noun e.g. "a" or "an", "the", this includes a plural form of that noun unless specifically stated. *Vice versa*, when the plural form of a noun is used it refers also to the singular form.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

According to the first aspect of the present invention, an ATP-dependent DNA helicase Q4 (RecQL4) mutein is provided, wherein the RecQL4 mutein differs from the wild-type sequence (SEQ ID NO:1) by one or more substitutions selected from P532S and R766Q.

SEQ ID NO:1 is the wild-type sequence of human RecQL4 and corresponds to the publicly available amino acid sequence NCBI Reference Sequence: NP004251.3 (Version: 6 January 2019). The amino acid sequence of SEQ ID NO:2 relates to the mutein differing from the wild-type sequence by a P532S substitution. The amino acid sequence of SEQ ID NO:3 relates to the mutein differing from the wild-type sequence by a R766Q substitution. When it comes to the individual amino acids of these amino acid sequences, it is noted that the individual amino acids may be modified. These modifications may be of natural origin (e.g. posttranslational) or introduced artificially such as glycosylation, di- or oligomerisation or modifications of the cysteine residues.

According to the second aspect, polynucleotides encoding the RecQL4 muteins according to the first aspect of the invention are provided.

SEQ ID NO: 4 relates to the genomic DNA encoding human RecQL4. SEQ ID NO:4 corresponds to the wild-type sequence which is also publicly available from NCBI Reference Sequence: NG_016430.1 (Version: 7 January 2019). SEQ ID NO:5 differs from SEQ ID NO:4 by the substitution of the codon CCC encoding proline at amino acid position 532 to the codon TCC encoding serine at this position. SEQ ID NO:6 differs from SEQ ID NO:4 by the substitution of the codon CGG encoding arginine at amino acid position 766 to the codon CAG encoding glutamine at this position.

The encoding polynucleotide may be selected from RNA, DNA or mixtures thereof. The DNA may be genomic DNA or cDNA. The polynucleotide may further be modified with respect to nuclease resistance. The polynucleotide may be fully or partially artificially synthesized by methods known in the art such as solid-phase synthesis. Site-directed mutagenesis may be used as well. The polynucleotide is then linked in an operative manner with a suitable promoter for expression in a suitable expression system. Suitable expression systems and promoters are also known in the art. The thus produced construct comprises at least a part of a vector. Said vector is capable of driving expression in a suitable expression system. For expression prokaryotic systems are contemplated as well as eukaryotic systems. In higher eukaryotic cells mammalian cells are preferred.

The expressed mutein according to the invention is subsequently purified using methods known in the art such as chromatographic methods or electrophoresis.

According to the third aspect, there is provided an in vitro method of determining the susceptibility for imidazotetrazine drug treatment of a human glioblastoma patient comprising
a) providing a nucleic acid sample from the human glioblastoma patient;
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of at least one amino acid substitution is indicative for the susceptibility of the human glioblastoma patient for imidazotetrazine drug treatment.

According a preferred embodiment, the imidazotetrazine drug is selected from dacarbazine, mitozolomide and temozolomide. A particularly preferred imidazotetrazine drug is temozolomide. The imidazotetrazine drugs are alkylating agents effective as anti-cancer compounds. In the examples, temozolomide has been used as drug. Due its close structural and functional relationships further imidazotetrazine compounds such as dacarbazine and mitozolomide are also encompassed by the present invention.

"A glioblastoma patient" (in the WHO stages: WHO IV grade glioma corresponds to glioblastoma) encompasses any subtype of glioblastoma patient. In the study by Verhaak et al., Cancer Cell 17 (2010), 98-110, four different subtypes of GBM patients have been identified, namely the Proneural, the Neural, the Classical and the Mesenchymal Subtype differing in their pattern of gene expression. The Classical GBM tumors are characterized by high levels of EGFR. In Proneural Tumors TP53 is significantly mutated. Further, the proneural tumors are also characterized by having the most frequent mutations in the IDH1 gene. Another gene, PDGFRA was also mutated and highly expressed. Further, the Mesenchymal subgroup contains the most frequent numbers of mutations in the NF1 tumor suppressor gene. Frequent mutations in the PTEN and TP53 tumor suppressor genes also occurred in this group. The Neural Group had mutations in many of the same genes as the other groups.

The term "susceptibility for treatment of a human glioblastoma patient" relates to the response of a human glioblastoma patients to the respective treatment, i.e. how susceptible this patient is for the treatment. As concluded by the present inventors, the human glioblastoma cells identified according to the invention suffer from a defect in the DNA repair system RecQL4, which should render corresponding human glioblastoma patients more susceptible to treatment using chemotherapeutic agents, in particular agents inducing DNA damages, compared to cells from glioblastoma patients, which do not possess a defect in this DNA repair system. On a general level, an increased susceptibility to drug treatment may be determined *in vitro* or *in vivo* by methods known in the art. For example, the susceptibility can be determined by a reduced glioblastoma cell survival and/or proliferation. Preferably, the reduction of cell proliferation of the tumor cells is monitored.

In order to obtain and/or provide a nucleic acid sample from the glioblastoma patient, a tissue sample or blood sample may be taken from said patient. It is, however, understood, that any other sample derived from the patient and from which a nucleic acid sample may be obtained such as sputum may also be used. Methods for obtaining a blood sample from a patient are known in the art. For example, a blood sample may be taken from a patient by using a sterile needle.

Subsequently, the DNA is extracted or purified from the sample prior to the subsequent mutational detection (SNP genotyping) analysis.

The SNP indicative for susceptibility is rs763278718 (CCC to TCC change for the codon at position 532 of SEQ ID NO: 1), and the further SNP indicative for susceptibility is rs931761657 (CGG to CAG for the codon at position 766 of SEQ ID NO:1).

Any method known in the art may be used for DNA extraction or purification. Suitable methods comprise *inter alia* steps such as centrifugation steps, precipitation steps, chromatography steps, dialyzing steps, heating steps, cooling steps and/or denaturation steps. For some embodiments, a certain DNA content in the sample may have to be reached. DNA content can be measured for example via UV spectrometry as described in the literature. Thus, DNA amplification may be useful prior to the SNP analysis step. Any method known in the art can be used for DNA amplification. The sample can thus be provided in a concentration and solution appropriate for the SNP analysis.

For the SNP genotyping analysis performed in step b), SNP-specific primers and/or probes, a primer extension reaction, SNP microarrays, restriction analysis and/or DNA-sequencing may be used. Reagents and methods for performing SNP genotyping analyses are known in the art.

In one embodiment of the invention, the SNP genotyping analysis performed in step b) of the method of the third aspect of the invention includes a PCR followed by restriction analysis. More specifically, after extraction of the DNA (e.g. from blood of the patient), two PCR amplifications are made to cover the exons 8 and 13 of the RecQL4 gene, separately.

In an alternative embodiment, the SNP genotyping analysis of step b) of the method of the third aspect of the invention is performed by DNA-sequencing. DNA sequencing usually employs a primer designed as flanking the region to be analysed together with labelled nucleotides in a PCR-like setup. By analysing the labels at the corresponding positions, it is possible to determine the sequence of DNA starting from the regions to which the primer is hybridising. Furthermore, it is possible to determine the genotype of an allele by sequencing since a peak corresponding to two different bases or a peak indicating an identical base at a certain position may be detected.

DNA-microarray techniques may also be used in step b), wherein the techniques are based on hybridisation events between the test-DNA and so-called "probes" immobilised on defined spots of a Microarray in a chamber. Today, such microarrays are routinely used to determine DNA-sequences even down to the level of a single base and thus for the detection of SNPs. This is possible by selecting the probes accordingly and using specific hybridisation conditions. The DNA may be labelled for detecting purposes. Routinely, probes covering the different sequences at the position of an SNP may be used in combination with corresponding controls; thus, also the genotype of the corresponding SNP may be analysed.

Further, real-time PCR methods may also be used in step b), wherein real-time PCR is based on the incorporation of double strand specific dyes into DNA while said DNA is amplified. Said dyes are detected only in case they are incorporated. Thus, the more DNA amplified, the higher the detection signal of the corresponding dye. By designing primers accordingly and/or by adding suited probe-nucleotides hybridising to a specific DNA-sequence only (which are able to discriminate between SNPs) and using specific hybridisation conditions, polymorphisms may be analysed.

Also, mass-spectrometry (MS) may be used in step b) of the present method. In MALDI-MS, a sample is mixed with a solution containing a matrix material and a drop of the liquid is placed on the surface of a probe. The matrix solution then e.g. co-crystallises with the biological sample and the probe is inserted into the mass spectrometer and laser energy is then directed to the probe surface where it absorbs and ionises the biological molecules without significantly fragmenting them.

In another embodiment of the method described herein the SNP genotyping analysis of step b) includes a combination of the above described methods.

If the result of step b) of the method of the third aspect of the present invention is the detection of one or more of said mutations, then this is indicative for the susceptibility of the human glioblastoma patient for imidazotetrazine drug treatment.

According to the fourth aspect, there is provided an *in vitro* method of determining the susceptibility for PARP inhibitor treatment of a human glioblastoma patient comprising
a) providing a nucleic acid sample from the human glioblastoma patient
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of at least one substitution is indicative for the susceptibility of the human glioblastoma patient for PARP inhibitor treatment.

Steps a) and b) may be performed as outlined above for steps a) and b) of the third aspect.

"PARP inhibitor" means any compound capable of inhibiting the enzyme poly(ADP-ribose) polymerase. The inhibitory activity can be determined by assays known in the art (see e.g. Weston, Victoria J., et al. Blood (2010): blood-2010; Nile, Donna L., et al. BMC cancer 16.1 (2016): 621; Yang, Xueli, et al. Oncology letters 9.2 (2015): 757-761).

According to a preferred embodiment, the PARP inhibitor is selected from the group consisting of olaparib, niraparib, rucaparib, talazoparib, veliparib, pamiparib, CEP 9722, E7016, iniparib, INO-1001 (3-aminobenazmide), E7449, AG-14361, UPF-1069 and A-966492. It can be more preferred that the PARP inhibitor is selected from the group consisting of olaparib, niraparib, rucaparib, talazoparib, veliparib, pamiparib, CEP 9722, E7016, E7449 and INO-1001 (3-aminobenazmide). A particularly preferred PARP inhibitor is olaparib.

PARP has a role in repair of single-stranded DNA (ssDNA) breaks. Olaparib, niraparib and rucaparib are approved in the treatment of ovarian cancer, while talazoparib was approved in 2018 in the US for breast cancer. Veliparib and pamiparib as well as CEP 9722 (see e.g. NCT number: NCT00920595 with the responsible party being Teva Pharmaceutical Industries (Cephalon) and Cephalon being study sponsor), E7016 (see e.g. NCT number: NCT01127178 with the responsible party and the study sponsor being Eisai Inc.) and E7449 (see e.g. NCT number: NCT01618136 with Eisai Limited being the responsible party and the study sponsor), have been or are presently being tested in clinical trials. Further PARP-inhibitors are known to the skilled person, e.g. from commercial sources where PARP-inhibitors are commercially available (e.g. from Selleckchem), and include iniparib, INO-1001 (3-aminobenazmide; see e.g. NCT number: NCT00271765 with Inotek Pharmaceuticals Corporation being study sponsor), AG-14361, UPF-1069 and A-966492.

Another aspect of the present invention relates to a kit for the analysis of a single nucleotide polymorphism indicative for the susceptibility for imidazotetrazine drug or PARP inhibitor treatment of a human glioblastoma patient, comprising at least one primer and/or probe for determining the genotype at rs763278718.

Yet another aspect of the present invention relates to a kit for the analysis of a single nucleotide polymorphism indicative for the susceptibility for imidazotetrazine drug or PARP inhibitor treatment of a human glioblastoma patient, comprising at least one primer and/or probe for determining the genotype at rs931761657.

The term "primer" as used herein denotes an oligonucleotide that acts as an initiation point of nucleotide synthesis under conditions in which synthesis of a primer extension product complementary to a nucleic acid strand is induced.
The term "probe" as used herein denotes an oligonucleotide that selectively hybridizes to a target nucleic acid under suitable conditions.

The primers and probes may be generated such that they are able to discriminate between wild-type allele or mutated allele of the position of the SNP to be analyzed, i.e. of rs763278718 and/or rs931761657. Methods for the design of sequence specific primers and probes are known in the art. Exemplary primer pairs (forward and reverse primer) may be selected from SEQ ID NOs: 7 and 8; SEQ ID Nos: 9 and 10; and SEQ ID Nos: 11 and 12.

According to a further aspect, an imidazotetrazine drug for use in the treatment of glioblastoma in a human patient is provided, wherein the patient has been identified in the method of the third aspect of the present invention.
The imidazotetrazine drug is preferably selected from dacarbazine, mitozolomide and temozolomide, preferably the imidazotetrazine drug is temozolomide. The imidazotetrazine may be formulated in a pharmaceutical composition. The pharmaceutical composition may be in the form of a tablet, capsule or in solution for parenteral administration.

Suitable dosage of the imidazotetrazine drug (preferably of temozolomide) ranges from about 50 to about 100 mg/m² PO/IV every day for about 40 days. Preferred is a dosage of about 75 mg/m² PO/IV every day for about 42 days. If necessary, the treatment may be continued, in particular up to about 49 days. The administration frequency may be once, twice or three times daily, wherein once a day is preferred.

According to a further aspect, a PARP inhibitor for use in the treatment of glioblastoma in a human patient is provided, wherein the human patient has been identified in the method according to the fourth aspect of the present invention.

Preferably, the PARP inhibitor is selected from the group consisting of olaparib, niraparib, rucaparib, talazoparib, veliparib, pamiparib, CEP 9722, E7016, iniparib, INO-1001 (3-aminobenazmide), E7449, AG-14361, UPF-1069 and A-966492. It can be more preferred that the PARP inhibitor is selected from the group consisting of olaparib, niraparib, rucaparib, talazoparib, veliparib, pamiparib, CEP 9722, E7016, E7449 and INO-1001 (3-aminobenazmide). A particularly preferred PARP inhibitor is olaparib. The PARP inhibitor may be formulated in a pharmaceutical composition. The pharmaceutical composition may be in the form of a tablet, capsule or in solution for parenteral administration.

Suitable dosage of the PARP inhibitor (preferably of olaparib) ranges from about 20 to about 200 mg/day, preferably from about 50 to about 150 mg/day. It can be preferred to administered these doses for about 4 weeks. The administration frequency may be once, twice or three times daily, wherein once daily is preferred.

The above described imidazotetrazine drug or PARP inhibitor can be applied as a monotherapy or in combination with known anti-cancer agents. Preferred known anti-cancer agents can be PCV, which is a combination of procarbazine, lomustine and vincristine.

The invention is further described in the following examples which are solely for the purpose of illustrating specific embodiments of the invention, and are also not to be construed as limiting the scope of the invention in any way.

### Examples

### Material and Methods

### Cell culture, treatments, cell function analyses

Standard methods were used. In particular, human glioma cell lines (established LN18, LN229, U87 and U251 cells from ATTC) were cultured in Dulbecco's-modified Eagle's medium (DMEM) supplemented with 10% fetal bovine serum FBS and antibiotics: 50 U/ml penicillin and 50 mg/ml streptomycin. IPIN (patient sample from the Psychiatric and Neurologic Institute, Warsaw) and WG4 (patient sample from Central Clinical Hospital of the MSWiA in Warsaw) cell lines (glioblastoma (i.e. WHO grade IV glioma) patient-derived primary cell cultures) were cultured in Dulbecco's modified Eagle medium (DMEM) with F12/GlutaMax, supplemented with 10% fetal bovine serum (Gibco Invitrogen) and 100 units/mL of penicillin, 100 µg/mL of streptomycin. NHA cells (Normal Human Astrocytes, purchased from Lonza [Walkersville, MD, USA]) were cultured in ABM Basal Medium (Lonza) supplemented with 3% fetal bovine serum, 1% 1-glutamine, 0.1% ascorbic acid, 0.1% human EGF, 0.1% gentamicin, and 0.0025% recombinant human insulin. Cells were grown in 6-well plates (for transfections, RNA and protein extract collection) or 96-well plates (for analysis of viability and proliferation). The cell cultures were maintained at 37°C in a humidified atmosphere of 5% CO₂ and 95% air.

### Preparation of RNA, DNA, protein extracts and Western Blot analysis

Standard methods were used. Thus, DNA and/or RNA from glioma cells was isolated using a TRI Reagent. Protein extracts were resolved by SDS-PAGE and electrotransfered onto a nitrocellulose membrane. A primary antibody was used for the Western Blot analysis. After blocking, the blots were washed and probed with the relevant secondary antibody. After that the immunoblots were re-probed with anti-B-actin antibody to ensure equal protein loading. Densitometric analysis was performed using Image software.

### RT-qPCR analysis

Total RNA from the cell cultures was extracted using High Pure RNA Isolation Kit and 1 µg of total RNA was used to synthesize cDNA by extension of oligo(dT)15 primers with SuperScript™ HI Reverse Transcriptase (Invitrogen). Real-time PCR amplifications were performed in duplicate on cDNA equivalent to 25 ng RNA in 10-µl reaction volume containing Fast Universal PCR Master Mix 2x (Applied) mixed with RECQL4 primers: Forward 5' GAGGAGGCCATCGAGACTTT' 3' (SEQ ID NO: 7); Reverse 5' GTATAGGTGGTCGCCAGCAG 3' (SEQ ID NO: 8) or GAPDH primers for normalization. The expression of each product was normalized to GAPDH and relative quantification of gene expression was determined with the comparative CT method.

### Ultra deep sequencing

Ultra deep sequencing was performed at the Nencki Institute, Poland, using Hiseq 1500 (in-house preparation). For ultra-deep sequencing, DNA was amplified with the following primers and conditions by qPCR:

| Primer | | name | Length | Tm | GC% | PRODUCT_LENGTH |
|---|---|---|---|---|---|---|
| F | CAAGTCCCTGTGCTACCAGC | RECQL4_H_F_seq | 20 | 60.67 | 60 | 153 |
| R | CACAAGTGCTGGTTCTTGGC | RECQL4_H_R_seq | 20 | 59.97 | 55 | |
| F | TGACCATCTGCCTGTCTTC | RECQL4_2_H_F_seq | 19 | 56.75 | 53 | 161 |
| R | CCAGCCCCATCCCAAAG | RECQL4_2_H_R_seq | 17 | 57.12 | 65 | |

Forward Primer 1 CAAGTCCCTGTGCTACCAGC (SEQ ID NO: 9)
Reverse Primer 1 CACAAGTGCTGGTTCTTGGC (SEQ ID NO: 10)
Forward Primer 2 TGACCATCTGCCTGTCTTC (SEQ ID NO: 11)
Reverse Primer 2 CCAGCCCCATCCCAAAG (SEQ ID NO: 12)

### Transfection with siRNA

siRNAs were introduced by AMAXA electroporation using two sets of specific and control siRNAs according to the manufacturer's instructions. Cells were transfected with 25 nM of RecQL4-specific siRNA or control siRNA (Dharmacon): ON-TARGETplus Human RECQL4 (9401) siRNA - Individual Dharmacon # J-010559-06-0002 - targets ORF & Non-Coding; ON-TARGETplus Human RECQL4 (9401) siRNA - Individual Dharmacon # J-010559-07-0002-targets ORF & Non-Coding, and two control siRNA (crtl, 2 ON-TARGETplus Non-targeting siRNA #1 - Individual Dharmacon # D-001810-01-05), which has no target in human RNA. Cells were collected for Western Blot 48 h post-transfection.

### CRISPR/Cas9

For generation of RECQL4 knock-out cell lines, CRISPR/Cas9-based genome editing technique was used. The CRISPRCLEAR™ Transfection ready KO kit (cat # ASK-7010), specific for human RECQL4, has been custom designed and developed by Applied Stem Cell. The kit included two items:
a) one validated gRNA, targeting exon 3 of human RECQL4 (CGCACTCTGAAGCTGACCAC, SEQ ID NO: 13) integrated onto expression vector; and
b) Cas9-Puro plasmid, co-expressing hSpCas9 and Puromycin resistance gene.

The glioma cell lines (LN18 and LN229) were co-transfected with both plasmids using Lipofectamine2000 (Invitrogen) for 48h, followed by puromycin (2 µg/ml) selection for 72h and incubation with puromycin-free media for additional 48h. On day 7 post-transfection, the reminding surviving cells were plated at low concentration for single cell colonies growth. Two clones for each cell line, LN18 (clone #6.4; clone #6.6) and LN229 (clone #6.5; clone #6.11), were selected for further studies, based on NGS ultra deep results, confirming deletions in exon 3 of the RECQL4 gene.

### RESULTS

### Identification of two novel RECQL4 mutations in glioblastoma patients

The specimen-collection as analyzed herein includes 200 WHO II, III and IV grade human glioma specimens obtained from: The Canadian Brain Tumor Tissue Bank (London Health Sciences Centre, Ontario CA), The Children's Memorial Health Institute, Warsaw (the study was approved by the Committee of Bioethics of the Children's Memorial Health Institute, #14/KBE/2012), The Institute of Psychiatry and Neurology, Warsaw (the study was approved by the Committee of Bioethics of The Institute of Psychiatry and Neurology, #8/2012), Scanmed S.A. St. Raphael Hospital in Cracow (the study was approved by the Committee of Bioethics of the District Medical Chamber in Cracow, #73/KBL/OIL/2015), and the Military Institute of Medicine in Warsaw (the study was approved by the Committee of Bioethics of the Military Institute of Medicine, #20/WIM/2016).

Targeted Next Generation Sequencing performed with the use of Illumina Hiseq 1500 platform in the Laboratory of Molecular Neurobiology revealed the occurrence of two somatic variants in the *RECQL4* gene in three out of 200 WHO II, III, and IV grade glioma patients specimens, wherein these three specimens are WHO grade IV glioma patients specimens.

In one out of 200 WHO II, III and IV grade glioma samples, the RECQL4 variant with the codon change Ccc/Tcc resulting in a missense variant (substitution from proline to serine at position 532 of SEQ ID NO: 1) was identified. In two out of 200 WHO II, III and IV grade glioma samples, the RECQL4 variant with the codon change cGg/cAg resulting in a missense variant (substitution from arginine to glutamine at position 766 of SEQ ID NO:1) was identified. In total 12 patients out of 200 WHO II, III and IV grade glioma patients were harboring possibly damaging/deleterious RECQL4 amino acid changes. Further details are given in

**Table 1:**

| **gene** | **sample** | **depth** | **variant type** | **amino acids** | **localization of mutation** |
|---|---|---|---|---|---|
| **RECQL4** | **GBM3210** | **155** | **missense_variant** | **R/Q** | **Chr8 exon 13** |
| **RECQL4** | **GBM3410** | **96** | **missense_variant** | **R/Q** | **Chr8 exon 13** |
| **RECQL4** | **CA348** | **91** | **missense_variant** | **P/S** | **Chr8 exon 8** |

The above three samples are derived from patients with WHO IV glioma, i.e. patients suffering from glioblastoma. 10 of the overall 12 patients out of 200 samples identified with RECQL4 changes were found in WHO grade IV samples.

Figure 2 shows a screenshot from Integrative Genome Browser (IGV) of a somatic variant in RECQL4 gene identified in GBM3210 (Glioblastoma multiforme) sample. A standard PCR was performed using High Fidelity DNA polymerase and primers specific for RECQL4 gene using the DNA isolated from low- and high-grade glioma samples (NGS). The variant presence was confirmed in the same sample by ultra-deep sequencing (UDS) using RECQL4 specific primers. In the upper panel each grey horizontal bar represents a sequencing read from Illumina Hiseq 1500 platform. Occurrence of T (Thymine) in a top sample is a somatic mutation, as it was not detected in the DNA from patient blood. The detected variants present in exon 8 and 13 have not been reported in glioma and other tumors until now. A previous report has shown that specific single nucleotide polymorphism (SNP) of RECQL4 gene introns may associate with glioblastoma patients outcome (Wibom et al., cited above).

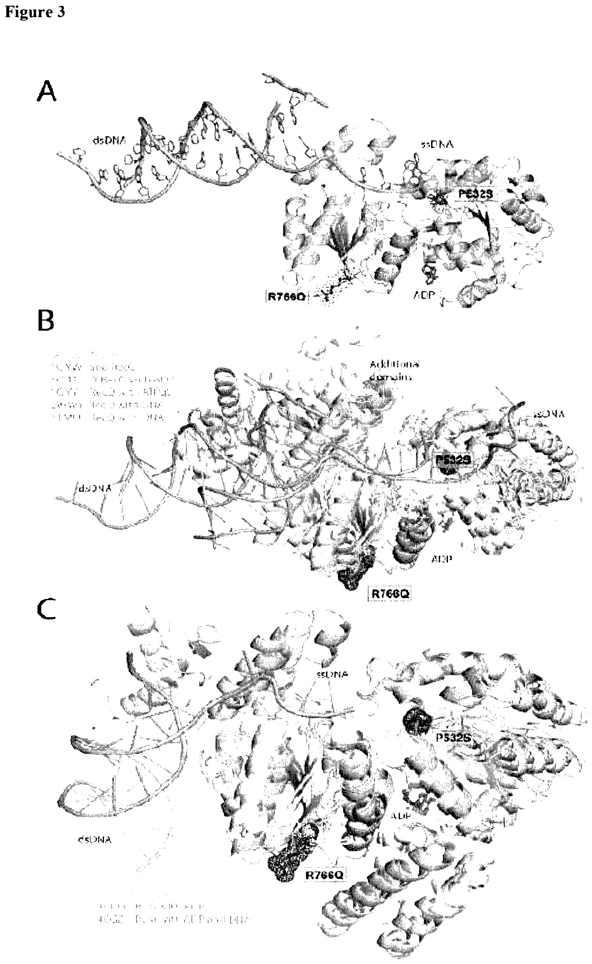

To establish an initial structure-function relationship and potential effects of variants on protein structure/function, preliminary structural homology modeling was performed. The analyses of the RecQL4 sequence were based on the crystal structure of its closest crystallized homologue, RecQ from *E. coli* (Bernstein et al., EMBO J. 22 (2003), 4910-4922) and show striking similarities in the helicase core domain (residues 102-48, Fig. 3A), which contains both identified mutations. Additional templates of related RecQ molecules from different species (e.g. *Deinococcus radiodurans* RecQ, see Chen et al., Biomed. Res. Int. (2014); 2014:342725) were used to assess the relative position of the mutants in relation to the known ADP, ATP and nucleic acid bindings sites (Fig. 3B and 3C). From these analyses it is obvious that P532 is located in close proximity of the bound ssDNA and the catalytic site for ATP hydrolysis, clearly indicating a functional relevance. In addition, a functional prediction server (PROVEAN) indicated that changing proline 532 (P532) into any other amino acid indeed leads to deleterious effects. Structural comparisons with human BLM helicases loaded with either ADP or ADP and DNA (Newman et al. Nucl. Acids Res. 43 (2015), 5221-5235) show that alterations in the position R766Q could also have some influence on protein function. These preliminary results indicate functional importance for both identified mutations that likely affect RecQL4 function and result in dysfunction or inactivity, respectively.

### RECQL 4 is overexpressed in glioblastoma

Real-time PCR amplification was performed in duplicates on cDNA equivalent to 25 ng RNA in 10-µl reaction volume containing Fast Universal PCR Master Mix 2x (Applied Biosystems, Darmstadt, Germany) and primers sets for: GAPDH and RECQL4 with the use of QuantStudio 12K Flex (Applied Biosystems, Life Technologies). The expression of a product was normalized to GAPDH, used as an internal reference gene. Relative quantification of gene expression was determined using the comparative CT method and is shown as a ratio of the target gene to GAPDH gene expression, calculated by 2^{-ΔΔCt}.

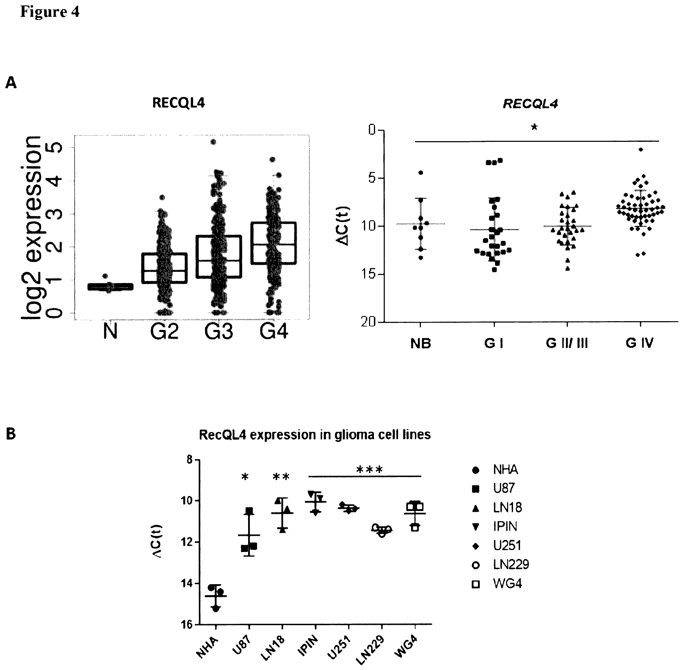

The levels of RECQL4 mRNA in normal brains (N) and gliomas of different grades (G2 = grade II, G3 = grade III, G4 = grade IV) are given in Fig. 4A, left side. In order to arrive at the expression levels, the TCGA database was used, which comprises more than 1000 glioma samples of the different grades and some matched normal brain samples. The RNAseq data were extracted from this database an analysis was performed to look at the expression level dependent on the type of cells. The right side of Fig. 4A shows the RECQL4 expression in a representative number of the 200 samples of different glioma samples as discussed above, namely as follows: 9 samples for "NB"; 25 samples for "GI"; 29 samples for "G II/III" and 50 samples for "G IV". The RECQL4 mRNA expression was analyzed as described above. It can be concluded that RECQL4 is significantly overexpressed in G IV (glioblastomas) versus normal (N) brain (mean +/- s.d.; T test). Fig. 4B shows the RECQL4 expression based on qPCR across different glioma cell lines of n=3 independent experiments, wherein NHA = normal human astrocytes; U87 and U251 = glioma WHO grade III; and LN18, LN229, IPIN and WG4 = glioblastoma cell lines (WHO grade IV).

### The expression of RECQL4 was knockdown in LN18 and U87 cells

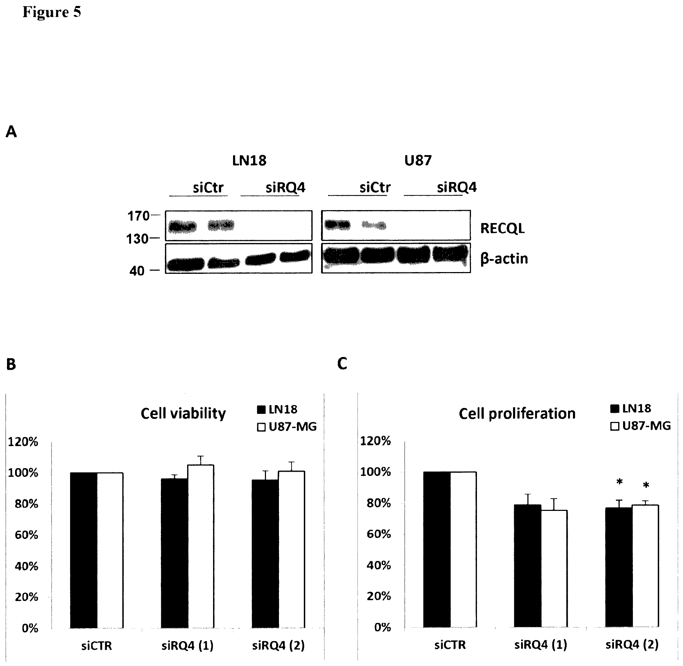

LN18 and U87 cells (1 x 10³ cells) were transfected by AMAXA electroporation with two different siRNAs. These studies indicate that the efficient knockdown of RECQL4 in two glioma cell lines results in a reduction of about 20-25% of cell proliferation (BrdU incorporation test) and does not have any significant effect on cell viability (MTT metabolism test), see Fig. 5: (A) RECQL4 was effectively knocked-down with siRNAs (by two different individual siRQ4) in LN18 and U87-MG glioma cells. Cell viability (B) and proliferation (C) were determined with MTT and BrdU assay, respectively. The results were normalized to cells transfected with siCTR and represent the mean ± SEM of n=4 independent experiments. Student's t-Test p<0.05.

### Depletion of RECQL4 sensitizes glioma cells to temozolomide

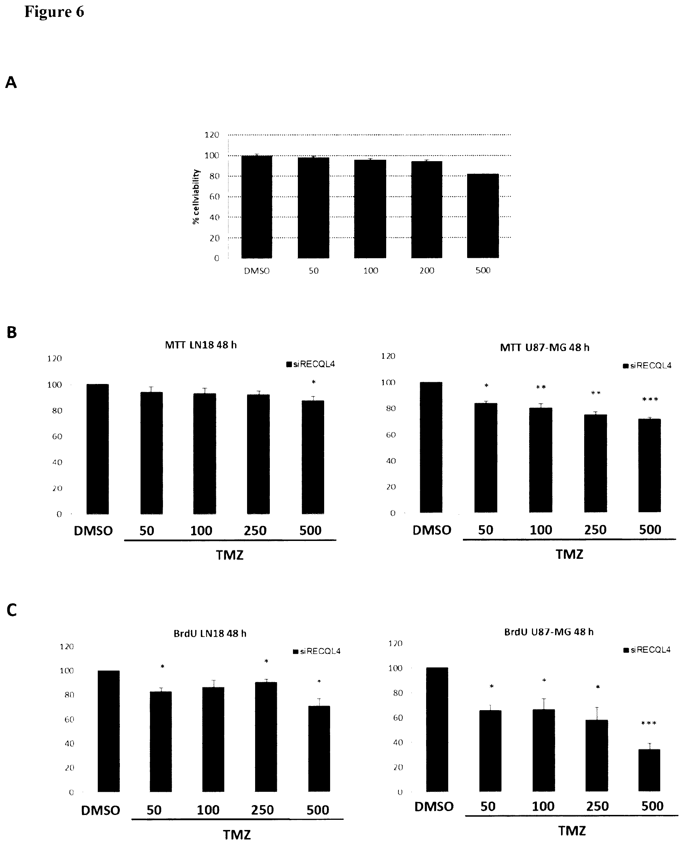

RECQL4-deficient cells from Rothmund-Thomson syndrome patients are hypersensitive to oxidative stress/damage and genotoxic agents. The inventors explored whether knockdown of RECQL4 in glioma cells (LN18 and U87 cells) would modulate cell viability or proliferation of tumor cells to temozolomide (TMZ), a clinically used chemotherapeutic. The effect of increasing concentrations of TMZ on viability of LN18 cells with wildtype RECQL4 are shown on the left side of Figure 6.

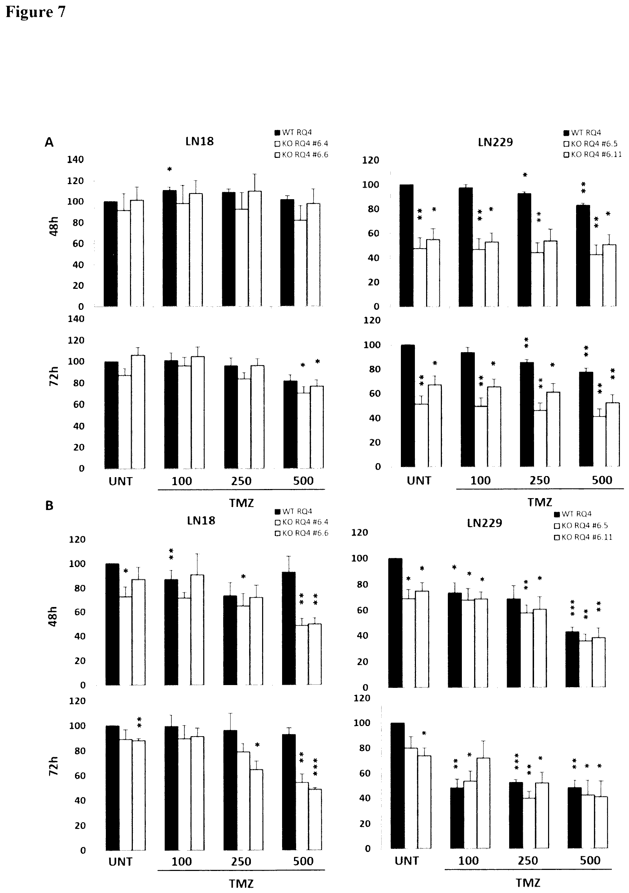

Further, the inventors explored whether stable knockout (mediated by CRISPR/Cas9) of RECQL4 in glioma cells would also modulate cell viability or proliferation of tumor cells to temozolomide (TMZ). The results are shown in Figure 7.

Stable knock-out of RECQL4 sensitizes glioma cells to temozolomide treatment. Glioma cell lines (LN18 and LN229) with stable knock-out of RECQL4 were exposed to temozolomide (TMZ) at multiple concentrations of the drug (100, 250, 500 µM) for 48 and 72h. The cells were then evaluated for (A) cell viability/ survival and (B) proliferation, with MTT and BrdU assays, respectively. The results were normalized to wild type cells (WT) with no drug treatment (H₂O as a vehicle control). The bars represent the mean ± SEM of n=4 independent experiments. One-way Anova analysis* p<0.05 ** p<0.01 *** p<0.001.

### Depletion of RECQL4 sensitizes glioma cells to the PARP inhibitor olaparib

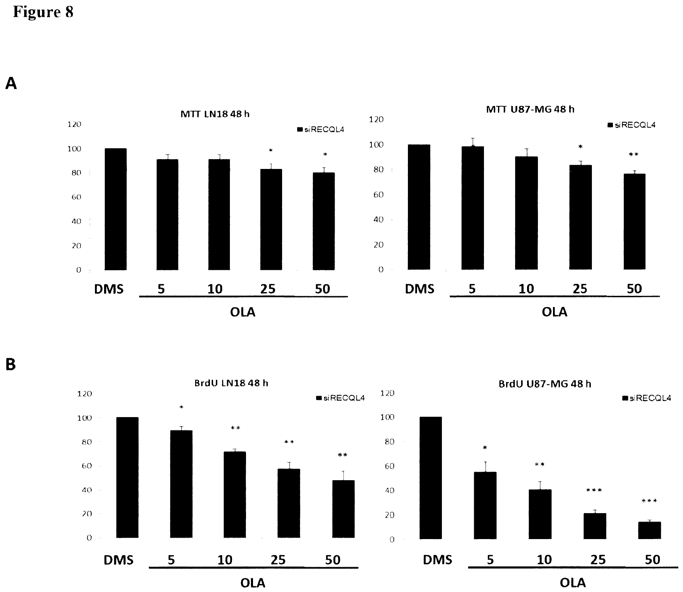

Olaparib (AZD-2281, trade name Lynparza) is an FDA-approved targeted therapy for ovarian cancer. The effects of olaparib on viability (MTT) and proliferation of BrdU on siRNA-treated glioma cells are shown in Figure 8. Glioma cells transfected with RECQL4 siRNA were left for 24 h, then were treated with DMSO (0.1%) or increasing concentrations of olaparib for the following 48 h (n=3; mean is.e.m., t test, *pv< 0.05, ** pv< 0.05, *** pv< 0.01. Olaparib (obtained from Merck) was dissolved in DMSO).

The results shown in Figure 8 demonstrate that Olaparib at concentrations >10 µM strongly impairs proliferation and growth of glioma tumor cells depleted on RECQL4. The effects are observed in two cell lines, wherein the cytostatic effects of Olaparib are stronger in U87MG cells. Olaparib at 25 µM reduces cell proliferation by 50% in LN18 cells and by 80% in U87MG cells.

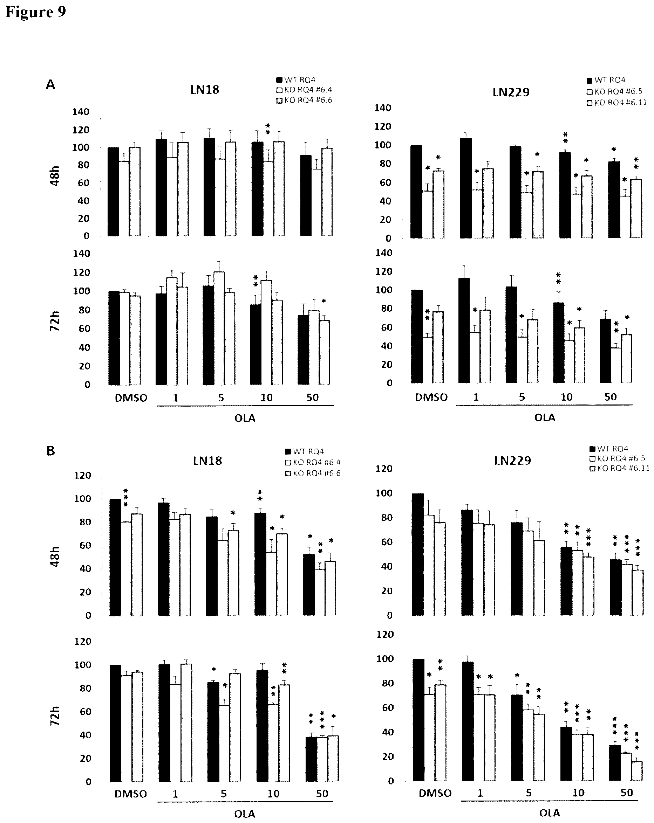

Figure 9 relates to the effects of Olaparib on viability and proliferation on RECQL4 stable knock-out glioma cells. Glioma cell lines (LN18 and LN229) with stable knock-out of RECQL4 were treated with Olaparib (OLA) at multiple concentrations of a drug (1, 5, 10, 50 µM) for 48 and 72h. The cells were then evaluated for (A) cell viability/ survival with MTT metabolism test and (B) cell proliferation with BrdU assay. The results were normalized to wild type cells (WT) with no drug treatment (H₂O as a vehicle control). The bars represent the mean ± SEM of n=4 independent experiments. One-way Anova analysis * p<0.05 ** p<0.01 *** p<0.001.

## Claims

1. An ATP-dependent DNA helicase Q4 (RecQL4) mutein, wherein the RecQL4 mutein differs from the wild-type sequence (SEQ ID NO:1) by one or more substitutions selected from P532S and R766Q of the wild-type human RecQL4 amino acid sequence of SEQ ID NO:1.

2. The RecQL4 mutein according to claim 1 comprising an amino acid sequence of SEQ ID NO: 2.

3. The RecQL4 mutein according to claim 1 comprising an amino acid sequence of SEQ ID NO:3.

4. A polynucleotide encoding the RecQL4 mutein of any one of claims 1 to 3.

5. The polynucleotide according to claim 4, wherein the polynucleotide comprises the sequence of SEQ ID NO:5 and encodes the RecQL4 mutein according to claim 2 or wherein the polynucleotide comprises the sequence of SEQ ID NO:6 and encodes the RecQL4 mutein according to claim 3.

6. An *in vitro* method of determining the susceptibility for imidazotetrazine drug treatment of a human glioblastoma patient comprising
a) providing a nucleic acid sample from the human glioblastoma patient;
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of one or more of said mutations is indicative for the susceptibility of the human glioblastoma patient for imidazotetrazine drug treatment.

7. The method of claim 6, wherein the imidazotetrazine drug is selected from the group consisting of dacarbazine, mitozolomide and temozolimide, preferably the imidazotetrazine drug is temozolomide.

8. An *in vitro* method of determining the susceptibility for PARP inhibitor treatment of a human glioblastoma patient comprising
a) providing a nucleic acid sample from the human glioblastoma patient;
b) detecting in the nucleic acid sample the presence of a mutation resulting in one or more amino acid substitutions selected from P532S and R766Q of the wild-type human RecQL4 amino acid sequence of SEQ ID NO:1;
wherein the detection of one or more of said mutations is indicative for the susceptibility of the human glioblastoma patient for PARP inhibitor treatment.

9. The *in vitro* method according to claim 8, wherein the PARP inhibitor is selected from the group consisting of olaparib, niraparib, rucaparib, talazoparib, veliparib, pamiparib, CEP 9722, E7016, iniparib, INO-1001 (3-aminobenazmide), E7449, AG-14361, UPF-1069 and A-966492, preferably wherein the PARP inhibitor is olaparib.

10. An imidazotetrazine drug for use in the treatment of glioblastoma in a human patient, wherein the patient has been identified in the method of any one of claim 6 or 7.

11. The imidatzotetrazine drug for use according to claim 10, wherein the imidazotetrazine drug is selected from the group consisting of dacarbazine, mitozolomide and temozolomide, preferably wherein the imidazotetrazine drug is temozolomide.

12. A PARP inhibitor for use in the treatment of glioblastoma in a human patient, wherein the human patient has been identified in the method of claim 8 or 9.

13. The PARP inhibitor for use according to claim 12, wherein the PARP inhibitor is selected from the group consisting of olaparib, niraparib, rucaparib, talazoparib, veliparib, pamiparib, CEP 9722, E7016, iniparib, INO-1001 (3-aminobenazmide), E7449, AG-14361, UPF-1069 and A-966492, preferably wherein the PARP inhibitor is olaparib.

14. A kit for the analysis of a single nucleotide polymorphism indicative for the susceptibility for imidazotetrazine drug or PARP inhibitor treatment of a human glioblastoma patient, comprising at least one primer and/or probe for determining the genotype at rs763278718.

15. A kit for the analysis of a single nucleotide polymorphism indicative for the susceptibility for imidazotetrazine drug or PARP inhibitor treatment of a human glioblastoma patient, comprising at least one primer and/or probe for determining the genotype at rs931761657.


SEQUENCE LISTING

<110>  Selvita S.A.

<120>  Novel RecQL4 muteins and personalized treatment of glioblastoma
       patients

<130>  S11767EP

<160>  13

<170>  PatentIn version 3.5

<210>  1
<211>  1190
<212>  PRT
<213>  Homo sapiens

<400>  1

Met Glu Arg Leu Arg Asp Val Arg Glu Arg Leu Gln Ala Trp Glu Arg
1               5                   10                  15

Ala Phe Arg Arg Gln Arg Gly Arg Arg Pro Ser Gln Asp Asp Val Glu
            20                  25                  30

Ala Ala Pro Glu Glu Thr Arg Ala Leu Tyr Arg Glu Tyr Arg Thr Leu
        35                  40                  45

Lys Arg Thr Thr Gly Gln Ala Gly Gly Gly Leu Arg Ser Ser Glu Ser
    50                  55                  60

Leu Pro Ala Ala Ala Glu Glu Ala Pro Glu Pro Arg Cys Trp Gly Pro
65                  70                  75                  80

His Leu Asn Arg Ala Ala Thr Lys Ser Pro Gln Ser Thr Pro Gly Arg
                85                  90                  95

Ser Arg Gln Gly Ser Val Pro Asp Tyr Gly Gln Arg Leu Lys Ala Asn
            100                 105                 110

Leu Lys Gly Thr Leu Gln Ala Gly Pro Ala Leu Gly Arg Arg Pro Trp
        115                 120                 125

Pro Leu Gly Arg Ala Ser Ser Lys Ala Ser Thr Pro Lys Pro Pro Gly
    130                 135                 140

Thr Gly Pro Val Pro Ser Phe Ala Glu Lys Val Ser Asp Glu Pro Pro
145                 150                 155                 160

Gln Leu Pro Glu Pro Gln Pro Arg Pro Gly Arg Leu Gln His Leu Gln
                165                 170                 175

Ala Ser Leu Ser Gln Arg Leu Gly Ser Leu Asp Pro Gly Trp Leu Gln
            180                 185                 190

Arg Cys His Ser Glu Val Pro Asp Phe Leu Gly Ala Pro Lys Ala Cys
        195                 200                 205

Arg Pro Asp Leu Gly Ser Glu Glu Ser Gln Leu Leu Ile Pro Gly Glu
    210                 215                 220

Ser Ala Val Leu Gly Pro Gly Ala Gly Ser Gln Gly Pro Glu Ala Ser
225                 230                 235                 240

Ala Phe Gln Glu Val Ser Ile Arg Val Gly Ser Pro Gln Pro Ser Ser
                245                 250                 255

Ser Gly Gly Glu Lys Arg Arg Trp Asn Glu Glu Pro Trp Glu Ser Pro
            260                 265                 270

Ala Gln Val Gln Gln Glu Ser Ser Gln Ala Gly Pro Pro Ser Glu Gly
        275                 280                 285

Ala Gly Ala Val Ala Val Glu Glu Asp Pro Pro Gly Glu Pro Val Gln
    290                 295                 300

Ala Gln Pro Pro Gln Pro Cys Ser Ser Pro Ser Asn Pro Arg Tyr His
305                 310                 315                 320

Gly Leu Ser Pro Ser Ser Gln Ala Arg Ala Gly Lys Ala Glu Gly Thr
                325                 330                 335

Ala Pro Leu His Ile Phe Pro Arg Leu Ala Arg His Asp Arg Gly Asn
            340                 345                 350

Tyr Val Arg Leu Asn Met Lys Gln Lys His Tyr Val Arg Gly Arg Ala
        355                 360                 365

Leu Arg Ser Arg Leu Leu Arg Lys Gln Ala Trp Lys Gln Lys Trp Arg
    370                 375                 380

Lys Lys Gly Glu Cys Phe Gly Gly Gly Gly Ala Thr Val Thr Thr Lys
385                 390                 395                 400

Glu Ser Cys Phe Leu Asn Glu Gln Phe Asp His Trp Ala Ala Gln Cys
                405                 410                 415

Pro Arg Pro Ala Ser Glu Glu Asp Thr Asp Ala Val Gly Pro Glu Pro
            420                 425                 430

Leu Val Pro Ser Pro Gln Pro Val Pro Glu Val Pro Ser Leu Asp Pro
        435                 440                 445

Thr Val Leu Pro Leu Tyr Ser Leu Gly Pro Ser Gly Gln Leu Ala Glu
    450                 455                 460

Thr Pro Ala Glu Val Phe Gln Ala Leu Glu Gln Leu Gly His Gln Ala
465                 470                 475                 480

Phe Arg Pro Gly Gln Glu Arg Ala Val Met Arg Ile Leu Ser Gly Ile
                485                 490                 495

Ser Thr Leu Leu Val Leu Pro Thr Gly Ala Gly Lys Ser Leu Cys Tyr
            500                 505                 510

Gln Leu Pro Ala Leu Leu Tyr Ser Arg Arg Ser Pro Cys Leu Thr Leu
        515                 520                 525

Val Val Ser Pro Leu Leu Ser Leu Met Asp Asp Gln Val Ser Gly Leu
    530                 535                 540

Pro Pro Cys Leu Lys Ala Ala Cys Ile His Ser Gly Met Thr Arg Lys
545                 550                 555                 560

Gln Arg Glu Ser Val Leu Gln Lys Ile Arg Ala Ala Gln Val His Val
                565                 570                 575

Leu Met Leu Thr Pro Glu Ala Leu Val Gly Ala Gly Gly Leu Pro Pro
            580                 585                 590

Ala Ala Gln Leu Pro Pro Val Ala Phe Ala Cys Ile Asp Glu Ala His
        595                 600                 605

Cys Leu Ser Gln Trp Ser His Asn Phe Arg Pro Cys Tyr Leu Arg Val
    610                 615                 620

Cys Lys Val Leu Arg Glu Arg Met Gly Val His Cys Phe Leu Gly Leu
625                 630                 635                 640

Thr Ala Thr Ala Thr Arg Arg Thr Ala Ser Asp Val Ala Gln His Leu
                645                 650                 655

Ala Val Ala Glu Glu Pro Asp Leu His Gly Pro Ala Pro Val Pro Thr
            660                 665                 670

Asn Leu His Leu Ser Val Ser Met Asp Arg Asp Thr Asp Gln Ala Leu

        675                 680                 685

Leu Thr Leu Leu Gln Gly Lys Arg Phe Gln Asn Leu Asp Ser Ile Ile
    690                 695                 700

Ile Tyr Cys Asn Arg Arg Glu Asp Thr Glu Arg Ile Ala Ala Leu Leu
705                 710                 715                 720

Arg Thr Cys Leu His Ala Ala Trp Val Pro Gly Ser Gly Gly Arg Ala
                725                 730                 735

Pro Lys Thr Thr Ala Glu Ala Tyr His Ala Gly Met Cys Ser Arg Glu
            740                 745                 750

Arg Arg Arg Val Gln Arg Ala Phe Met Gln Gly Gln Leu Arg Val Val
        755                 760                 765

Val Ala Thr Val Ala Phe Gly Met Gly Leu Asp Arg Pro Asp Val Arg
    770                 775                 780

Ala Val Leu His Leu Gly Leu Pro Pro Ser Phe Glu Ser Tyr Val Gln
785                 790                 795                 800

Ala Val Gly Arg Ala Gly Arg Asp Gly Gln Pro Ala His Cys His Leu
                805                 810                 815

Phe Leu Gln Pro Gln Gly Glu Asp Leu Arg Glu Leu Arg Arg His Val
            820                 825                 830

His Ala Asp Ser Thr Asp Phe Leu Ala Val Lys Arg Leu Val Gln Arg
        835                 840                 845

Val Phe Pro Ala Cys Thr Cys Thr Cys Thr Arg Pro Pro Ser Glu Gln
    850                 855                 860

Glu Gly Ala Val Gly Gly Glu Arg Pro Val Pro Lys Tyr Pro Pro Gln
865                 870                 875                 880

Glu Ala Glu Gln Leu Ser His Gln Ala Ala Pro Gly Pro Arg Arg Val
                885                 890                 895

Cys Met Gly His Glu Arg Ala Leu Pro Ile Gln Leu Thr Val Gln Ala
            900                 905                 910

Leu Asp Met Pro Glu Glu Ala Ile Glu Thr Leu Leu Cys Tyr Leu Glu
        915                 920                 925

Leu His Pro His His Trp Leu Glu Leu Leu Ala Thr Thr Tyr Thr His
    930                 935                 940

Cys Arg Leu Asn Cys Pro Gly Gly Pro Ala Gln Leu Gln Ala Leu Ala
945                 950                 955                 960

His Arg Cys Pro Pro Leu Ala Val Cys Leu Ala Gln Gln Leu Pro Glu
                965                 970                 975

Asp Pro Gly Gln Gly Ser Ser Ser Val Glu Phe Asp Met Val Lys Leu
            980                 985                 990

Val Asp Ser Met Gly Trp Glu Leu  Ala Ser Val Arg Arg  Ala Leu Cys
        995                 1000                 1005

Gln Leu  Gln Trp Asp His Glu  Pro Arg Thr Gly Val  Arg Arg Gly
    1010                 1015                 1020

Thr Gly  Val Leu Val Glu Phe  Ser Glu Leu Ala Phe  His Leu Arg
    1025                 1030                 1035

Ser Pro  Gly Asp Leu Thr Ala  Glu Glu Lys Asp Gln  Ile Cys Asp
    1040                 1045                 1050

Phe Leu  Tyr Gly Arg Val Gln  Ala Arg Glu Arg Gln  Ala Leu Ala
    1055                 1060                 1065

Arg Leu  Arg Arg Thr Phe Gln  Ala Phe His Ser Val  Ala Phe Pro
    1070                 1075                 1080

Ser Cys  Gly Pro Cys Leu Glu  Gln Gln Asp Glu Glu  Arg Ser Thr
    1085                 1090                 1095

Arg Leu  Lys Asp Leu Leu Gly  Arg Tyr Phe Glu Glu  Glu Glu Gly
    1100                 1105                 1110

Gln Glu  Pro Gly Gly Met Glu  Asp Ala Gln Gly Pro  Glu Pro Gly
    1115                 1120                 1125

Gln Ala  Arg Leu Gln Asp Trp  Glu Asp Gln Val Arg  Cys Asp Ile
    1130                 1135                 1140

Arg Gln  Phe Leu Ser Leu Arg  Pro Glu Glu Lys Phe  Ser Ser Arg
    1145                 1150                 1155

Ala Val  Ala Arg Ile Phe His  Gly Ile Gly Ser Pro  Cys Tyr Pro
    1160                 1165                 1170

Ala Gln Val Tyr Gly Gln Asp Arg Arg Phe Trp Arg Lys Tyr Leu
    1175                1180                1185

His Leu
    1190

<210> 2
<211> 1190
<212> PRT
<213> Homo sapiens

<400> 2

Met Glu Arg Leu Arg Asp Val Arg Glu Arg Leu Gln Ala Trp Glu Arg
1               5                   10                  15

Ala Phe Arg Arg Gln Arg Gly Arg Arg Pro Ser Gln Asp Asp Val Glu
            20                  25                  30

Ala Ala Pro Glu Glu Thr Arg Ala Leu Tyr Arg Glu Tyr Arg Thr Leu
        35                  40                  45

Lys Arg Thr Thr Gly Gln Ala Gly Gly Gly Leu Arg Ser Ser Glu Ser
    50                  55                  60

Leu Pro Ala Ala Ala Glu Glu Ala Pro Glu Pro Arg Cys Trp Gly Pro
65                  70                  75                  80

His Leu Asn Arg Ala Ala Thr Lys Ser Pro Gln Ser Thr Pro Gly Arg
                85                  90                  95

Ser Arg Gln Gly Ser Val Pro Asp Tyr Gly Gln Arg Leu Lys Ala Asn
            100                 105                 110

Leu Lys Gly Thr Leu Gln Ala Gly Pro Ala Leu Gly Arg Arg Pro Trp
        115                 120                 125

Pro Leu Gly Arg Ala Ser Ser Lys Ala Ser Thr Pro Lys Pro Pro Gly
    130                 135                 140

Thr Gly Pro Val Pro Ser Phe Ala Glu Lys Val Ser Asp Glu Pro Pro
145                 150                 155                 160

Gln Leu Pro Glu Pro Gln Pro Arg Pro Gly Arg Leu Gln His Leu Gln
                165                 170                 175

Ala Ser Leu Ser Gln Arg Leu Gly Ser Leu Asp Pro Gly Trp Leu Gln
            180                 185                 190

Arg Cys His Ser Glu Val Pro Asp Phe Leu Gly Ala Pro Lys Ala Cys
        195                 200                 205

Arg Pro Asp Leu Gly Ser Glu Glu Ser Gln Leu Leu Ile Pro Gly Glu
    210                 215                 220

Ser Ala Val Leu Gly Pro Gly Ala Gly Ser Gln Gly Pro Glu Ala Ser
225                 230                 235                 240

Ala Phe Gln Glu Val Ser Ile Arg Val Gly Ser Pro Gln Pro Ser Ser
                245                 250                 255

Ser Gly Gly Glu Lys Arg Arg Trp Asn Glu Glu Pro Trp Glu Ser Pro
            260                 265                 270

Ala Gln Val Gln Gln Glu Ser Ser Gln Ala Gly Pro Pro Ser Glu Gly
        275                 280                 285

Ala Gly Ala Val Ala Val Glu Glu Asp Pro Pro Gly Glu Pro Val Gln
    290                 295                 300

Ala Gln Pro Pro Gln Pro Cys Ser Ser Pro Ser Asn Pro Arg Tyr His
305                 310                 315                 320

Gly Leu Ser Pro Ser Ser Gln Ala Arg Ala Gly Lys Ala Glu Gly Thr
                325                 330                 335

Ala Pro Leu His Ile Phe Pro Arg Leu Ala Arg His Asp Arg Gly Asn
            340                 345                 350

Tyr Val Arg Leu Asn Met Lys Gln Lys His Tyr Val Arg Gly Arg Ala
        355                 360                 365

Leu Arg Ser Arg Leu Leu Arg Lys Gln Ala Trp Lys Gln Lys Trp Arg
    370                 375                 380

Lys Lys Gly Glu Cys Phe Gly Gly Gly Gly Ala Thr Val Thr Thr Lys
385                 390                 395                 400

Glu Ser Cys Phe Leu Asn Glu Gln Phe Asp His Trp Ala Ala Gln Cys
                405                 410                 415

Pro Arg Pro Ala Ser Glu Glu Asp Thr Asp Ala Val Gly Pro Glu Pro
            420                 425                 430

Leu Val Pro Ser Pro Gln Pro Val Pro Glu Val Pro Ser Leu Asp Pro
        435                 440                 445

Thr Val Leu Pro Leu Tyr Ser Leu Gly Pro Ser Gly Gln Leu Ala Glu
    450                 455                 460

Thr Pro Ala Glu Val Phe Gln Ala Leu Glu Gln Leu Gly His Gln Ala
465                 470                 475                 480

Phe Arg Pro Gly Gln Glu Arg Ala Val Met Arg Ile Leu Ser Gly Ile
                485                 490                 495

Ser Thr Leu Leu Val Leu Pro Thr Gly Ala Gly Lys Ser Leu Cys Tyr
            500                 505                 510

Gln Leu Pro Ala Leu Leu Tyr Ser Arg Arg Ser Pro Cys Leu Thr Leu
        515                 520                 525

Val Val Ser Ser Leu Leu Ser Leu Met Asp Asp Gln Val Ser Gly Leu
    530                 535                 540

Pro Pro Cys Leu Lys Ala Ala Cys Ile His Ser Gly Met Thr Arg Lys
545                 550                 555                 560

Gln Arg Glu Ser Val Leu Gln Lys Ile Arg Ala Ala Gln Val His Val
                565                 570                 575

Leu Met Leu Thr Pro Glu Ala Leu Val Gly Ala Gly Gly Leu Pro Pro
            580                 585                 590

Ala Ala Gln Leu Pro Pro Val Ala Phe Ala Cys Ile Asp Glu Ala His
        595                 600                 605

Cys Leu Ser Gln Trp Ser His Asn Phe Arg Pro Cys Tyr Leu Arg Val
    610                 615                 620

Cys Lys Val Leu Arg Glu Arg Met Gly Val His Cys Phe Leu Gly Leu
625                 630                 635                 640

Thr Ala Thr Ala Thr Arg Arg Thr Ala Ser Asp Val Ala Gln His Leu
                645                 650                 655

Ala Val Ala Glu Glu Pro Asp Leu His Gly Pro Ala Pro Val Pro Thr
            660                 665                 670

Asn Leu His Leu Ser Val Ser Met Asp Arg Asp Thr Asp Gln Ala Leu
        675                 680                 685

Leu Thr Leu Leu Gln Gly Lys Arg Phe Gln Asn Leu Asp Ser Ile Ile

690                 695                 700

Ile Tyr Cys Asn Arg Arg Glu Asp Thr Glu Arg Ile Ala Ala Leu Leu
705                 710                 715                 720

Arg Thr Cys Leu His Ala Ala Trp Val Pro Gly Ser Gly Gly Arg Ala
                725                 730                 735

Pro Lys Thr Thr Ala Glu Ala Tyr His Ala Gly Met Cys Ser Arg Glu
            740                 745                 750

Arg Arg Arg Val Gln Arg Ala Phe Met Gln Gly Gln Leu Arg Val Val
        755                 760                 765

Val Ala Thr Val Ala Phe Gly Met Gly Leu Asp Arg Pro Asp Val Arg
    770                 775                 780

Ala Val Leu His Leu Gly Leu Pro Pro Ser Phe Glu Ser Tyr Val Gln
785                 790                 795                 800

Ala Val Gly Arg Ala Gly Arg Asp Gly Gln Pro Ala His Cys His Leu
                805                 810                 815

Phe Leu Gln Pro Gln Gly Glu Asp Leu Arg Glu Leu Arg Arg His Val
            820                 825                 830

His Ala Asp Ser Thr Asp Phe Leu Ala Val Lys Arg Leu Val Gln Arg
        835                 840                 845

Val Phe Pro Ala Cys Thr Cys Thr Cys Thr Arg Pro Pro Ser Glu Gln
    850                 855                 860

Glu Gly Ala Val Gly Gly Glu Arg Pro Val Pro Lys Tyr Pro Pro Gln
865                 870                 875                 880

Glu Ala Glu Gln Leu Ser His Gln Ala Ala Pro Gly Pro Arg Arg Val
                885                 890                 895

Cys Met Gly His Glu Arg Ala Leu Pro Ile Gln Leu Thr Val Gln Ala
            900                 905                 910

Leu Asp Met Pro Glu Glu Ala Ile Glu Thr Leu Leu Cys Tyr Leu Glu
        915                 920                 925

Leu His Pro His His Trp Leu Glu Leu Leu Ala Thr Thr Tyr Thr His
    930                 935                 940

Cys Arg Leu Asn Cys Pro Gly Gly Pro Ala Gln Leu Gln Ala Leu Ala
945                 950                 955                 960

His Arg Cys Pro Pro Leu Ala Val Cys Leu Ala Gln Gln Leu Pro Glu
                965                 970                 975

Asp Pro Gly Gln Gly Ser Ser Ser Val Glu Phe Asp Met Val Lys Leu
            980                 985                 990

Val Asp Ser Met Gly Trp Glu Leu  Ala Ser Val Arg Arg  Ala Leu Cys
        995                 1000                 1005

Gln Leu  Gln Trp Asp His Glu  Pro Arg Thr Gly Val  Arg Arg Gly
    1010                 1015                 1020

Thr Gly  Val Leu Val Glu Phe  Ser Glu Leu Ala Phe  His Leu Arg
    1025                 1030                 1035

Ser Pro  Gly Asp Leu Thr Ala  Glu Glu Lys Asp Gln  Ile Cys Asp
    1040                 1045                 1050

Phe Leu  Tyr Gly Arg Val Gln  Ala Arg Glu Arg Gln  Ala Leu Ala
    1055                 1060                 1065

Arg Leu  Arg Arg Thr Phe Gln  Ala Phe His Ser Val  Ala Phe Pro
    1070                 1075                 1080

Ser Cys  Gly Pro Cys Leu Glu  Gln Gln Asp Glu Glu  Arg Ser Thr
    1085                 1090                 1095

Arg Leu  Lys Asp Leu Leu Gly  Arg Tyr Phe Glu Glu  Glu Glu Gly
    1100                 1105                 1110

Gln Glu  Pro Gly Gly Met Glu  Asp Ala Gln Gly Pro  Glu Pro Gly
    1115                 1120                 1125

Gln Ala  Arg Leu Gln Asp Trp  Glu Asp Gln Val Arg  Cys Asp Ile
    1130                 1135                 1140

Arg Gln  Phe Leu Ser Leu Arg  Pro Glu Glu Lys Phe  Ser Ser Arg
    1145                 1150                 1155

Ala Val  Ala Arg Ile Phe His  Gly Ile Gly Ser Pro  Cys Tyr Pro
    1160                 1165                 1170

Ala Gln  Val Tyr Gly Gln Asp  Arg Arg Phe Trp Arg  Lys Tyr Leu
    1175                 1180                 1185

His Leu
    1190

<210>  3
<211>  1190
<212>  PRT
<213>  Homo sapiens

<400>  3

Met Glu Arg Leu Arg Asp Val Arg Glu Arg Leu Gln Ala Trp Glu Arg
1               5                   10                  15

Ala Phe Arg Arg Gln Arg Gly Arg Arg Pro Ser Gln Asp Asp Val Glu
            20                  25                  30

Ala Ala Pro Glu Glu Thr Arg Ala Leu Tyr Arg Glu Tyr Arg Thr Leu
        35                  40                  45

Lys Arg Thr Thr Gly Gln Ala Gly Gly Gly Leu Arg Ser Ser Glu Ser
    50                  55                  60

Leu Pro Ala Ala Ala Glu Glu Ala Pro Glu Pro Arg Cys Trp Gly Pro
65                  70                  75                  80

His Leu Asn Arg Ala Ala Thr Lys Ser Pro Gln Ser Thr Pro Gly Arg
                85                  90                  95

Ser Arg Gln Gly Ser Val Pro Asp Tyr Gly Gln Arg Leu Lys Ala Asn
            100                 105                 110

Leu Lys Gly Thr Leu Gln Ala Gly Pro Ala Leu Gly Arg Arg Pro Trp
        115                 120                 125

Pro Leu Gly Arg Ala Ser Ser Lys Ala Ser Thr Pro Lys Pro Pro Gly
    130                 135                 140

Thr Gly Pro Val Pro Ser Phe Ala Glu Lys Val Ser Asp Glu Pro Pro
145                 150                 155                 160

Gln Leu Pro Glu Pro Gln Pro Arg Pro Gly Arg Leu Gln His Leu Gln
                165                 170                 175

Ala Ser Leu Ser Gln Arg Leu Gly Ser Leu Asp Pro Gly Trp Leu Gln
            180                 185                 190

Arg Cys His Ser Glu Val Pro Asp Phe Leu Gly Ala Pro Lys Ala Cys
        195                 200                 205

Arg Pro Asp Leu Gly Ser Glu Glu Ser Gln Leu Leu Ile Pro Gly Glu
    210                 215                 220

Ser Ala Val Leu Gly Pro Gly Ala Gly Ser Gln Gly Pro Glu Ala Ser
225                 230                 235                 240

Ala Phe Gln Glu Val Ser Ile Arg Val Gly Ser Pro Gln Pro Ser Ser
                245                 250                 255

Ser Gly Gly Glu Lys Arg Arg Trp Asn Glu Glu Pro Trp Glu Ser Pro
            260                 265                 270

Ala Gln Val Gln Gln Glu Ser Ser Gln Ala Gly Pro Pro Ser Glu Gly
        275                 280                 285

Ala Gly Ala Val Ala Val Glu Glu Asp Pro Pro Gly Glu Pro Val Gln
    290                 295                 300

Ala Gln Pro Pro Gln Pro Cys Ser Ser Pro Ser Asn Pro Arg Tyr His
305                 310                 315                 320

Gly Leu Ser Pro Ser Ser Gln Ala Arg Ala Gly Lys Ala Glu Gly Thr
                325                 330                 335

Ala Pro Leu His Ile Phe Pro Arg Leu Ala Arg His Asp Arg Gly Asn
            340                 345                 350

Tyr Val Arg Leu Asn Met Lys Gln Lys His Tyr Val Arg Gly Arg Ala
        355                 360                 365

Leu Arg Ser Arg Leu Leu Arg Lys Gln Ala Trp Lys Gln Lys Trp Arg
    370                 375                 380

Lys Lys Gly Glu Cys Phe Gly Gly Gly Gly Ala Thr Val Thr Thr Lys
385                 390                 395                 400

Glu Ser Cys Phe Leu Asn Glu Gln Phe Asp His Trp Ala Ala Gln Cys
                405                 410                 415

Pro Arg Pro Ala Ser Glu Glu Asp Thr Asp Ala Val Gly Pro Glu Pro
            420                 425                 430

Leu Val Pro Ser Pro Gln Pro Val Pro Glu Val Pro Ser Leu Asp Pro
        435                 440                 445

Thr Val Leu Pro Leu Tyr Ser Leu Gly Pro Ser Gly Gln Leu Ala Glu
    450                 455                 460

Thr Pro Ala Glu Val Phe Gln Ala Leu Glu Gln Leu Gly His Gln Ala
465                 470                 475                 480

Phe Arg Pro Gly Gln Glu Arg Ala Val Met Arg Ile Leu Ser Gly Ile
                485                 490                 495

Ser Thr Leu Leu Val Leu Pro Thr Gly Ala Gly Lys Ser Leu Cys Tyr
            500                 505                 510

Gln Leu Pro Ala Leu Leu Tyr Ser Arg Arg Ser Pro Cys Leu Thr Leu
        515                 520                 525

Val Val Ser Pro Leu Leu Ser Leu Met Asp Asp Gln Val Ser Gly Leu
    530                 535                 540

Pro Pro Cys Leu Lys Ala Ala Cys Ile His Ser Gly Met Thr Arg Lys
545                 550                 555                 560

Gln Arg Glu Ser Val Leu Gln Lys Ile Arg Ala Ala Gln Val His Val
                565                 570                 575

Leu Met Leu Thr Pro Glu Ala Leu Val Gly Ala Gly Gly Leu Pro Pro
            580                 585                 590

Ala Ala Gln Leu Pro Pro Val Ala Phe Ala Cys Ile Asp Glu Ala His
        595                 600                 605

Cys Leu Ser Gln Trp Ser His Asn Phe Arg Pro Cys Tyr Leu Arg Val
    610                 615                 620

Cys Lys Val Leu Arg Glu Arg Met Gly Val His Cys Phe Leu Gly Leu
625                 630                 635                 640

Thr Ala Thr Ala Thr Arg Arg Thr Ala Ser Asp Val Ala Gln His Leu
                645                 650                 655

Ala Val Ala Glu Glu Pro Asp Leu His Gly Pro Ala Pro Val Pro Thr
            660                 665                 670

Asn Leu His Leu Ser Val Ser Met Asp Arg Asp Thr Asp Gln Ala Leu
        675                 680                 685

Leu Thr Leu Leu Gln Gly Lys Arg Phe Gln Asn Leu Asp Ser Ile Ile
    690                 695                 700

Ile Tyr Cys Asn Arg Arg Glu Asp Thr Glu Arg Ile Ala Ala Leu Leu

705                 710                 715                 720

Arg Thr Cys Leu His Ala Ala Trp Val Pro Gly Ser Gly Gly Arg Ala
                725                 730                 735

Pro Lys Thr Thr Ala Glu Ala Tyr His Ala Gly Met Cys Ser Arg Glu
            740                 745                 750

Arg Arg Arg Val Gln Arg Ala Phe Met Gln Gly Gln Leu Gln Val Val
        755                 760                 765

Val Ala Thr Val Ala Phe Gly Met Gly Leu Asp Arg Pro Asp Val Arg
    770                 775                 780

Ala Val Leu His Leu Gly Leu Pro Pro Ser Phe Glu Ser Tyr Val Gln
785                 790                 795                 800

Ala Val Gly Arg Ala Gly Arg Asp Gly Gln Pro Ala His Cys His Leu
                805                 810                 815

Phe Leu Gln Pro Gln Gly Glu Asp Leu Arg Glu Leu Arg Arg His Val
            820                 825                 830

His Ala Asp Ser Thr Asp Phe Leu Ala Val Lys Arg Leu Val Gln Arg
        835                 840                 845

Val Phe Pro Ala Cys Thr Cys Thr Cys Thr Arg Pro Pro Ser Glu Gln
    850                 855                 860

Glu Gly Ala Val Gly Gly Glu Arg Pro Val Pro Lys Tyr Pro Pro Gln
865                 870                 875                 880

Glu Ala Glu Gln Leu Ser His Gln Ala Ala Pro Gly Pro Arg Arg Val
                885                 890                 895

Cys Met Gly His Glu Arg Ala Leu Pro Ile Gln Leu Thr Val Gln Ala
            900                 905                 910

Leu Asp Met Pro Glu Glu Ala Ile Glu Thr Leu Leu Cys Tyr Leu Glu
        915                 920                 925

Leu His Pro His His Trp Leu Glu Leu Leu Ala Thr Thr Tyr Thr His
    930                 935                 940

Cys Arg Leu Asn Cys Pro Gly Gly Pro Ala Gln Leu Gln Ala Leu Ala
945                 950                 955                 960

His Arg Cys Pro Pro Leu Ala Val Cys Leu Ala Gln Gln Leu Pro Glu
                965                 970                 975

Asp Pro Gly Gln Gly Ser Ser Ser Val Glu Phe Asp Met Val Lys Leu
            980                 985                 990

Val Asp Ser Met Gly Trp Glu Leu  Ala Ser Val Arg Arg  Ala Leu Cys
        995                 1000                 1005

Gln Leu  Gln Trp Asp His Glu  Pro Arg Thr Gly Val  Arg Arg Gly
    1010                 1015                 1020

Thr Gly  Val Leu Val Glu Phe  Ser Glu Leu Ala Phe  His Leu Arg
    1025                 1030                 1035

Ser Pro  Gly Asp Leu Thr Ala  Glu Glu Lys Asp Gln  Ile Cys Asp
    1040                 1045                 1050

Phe Leu  Tyr Gly Arg Val Gln  Ala Arg Glu Arg Gln  Ala Leu Ala
    1055                 1060                 1065

Arg Leu  Arg Arg Thr Phe Gln  Ala Phe His Ser Val  Ala Phe Pro
    1070                 1075                 1080

Ser Cys  Gly Pro Cys Leu Glu  Gln Gln Asp Glu Glu  Arg Ser Thr
    1085                 1090                 1095

Arg Leu  Lys Asp Leu Leu Gly  Arg Tyr Phe Glu Glu  Glu Glu Gly
    1100                 1105                 1110

Gln Glu  Pro Gly Gly Met Glu  Asp Ala Gln Gly Pro  Glu Pro Gly
    1115                 1120                 1125

Gln Ala  Arg Leu Gln Asp Trp  Glu Asp Gln Val Arg  Cys Asp Ile
    1130                 1135                 1140

Arg Gln  Phe Leu Ser Leu Arg  Pro Glu Glu Lys Phe  Ser Ser Arg
    1145                 1150                 1155

Ala Val  Ala Arg Ile Phe His  Gly Ile Gly Ser Pro  Cys Tyr Pro
    1160                 1165                 1170

Ala Gln  Val Tyr Gly Gln Asp  Arg Arg Phe Trp Arg  Lys Tyr Leu
    1175                 1180                 1185

His Leu
    1190

<210>  4
<211>  13543
<212>  DNA
<213>  Homo sapiens

<400>  4
cgccctgggc gtggccacac agacggccat tgcggcggcc atcgcgctgc tggcgctcac      60

ggcgctgctc ctggtcgcca tgatctgtcg ccggcgccgc aggcgaaaaa aggcgcgggg     120

gcctccgggg gagggagcgc tgttcgtcaa cgactacttg gacggcccct gtacgttcgc     180

acagctagag gagctccgcg acgagcgcgg ccacgagatg ttcgtcatca accgctccaa     240

gccgctcttc gccgagggtc cggcggaggc gcccgctgac tgcggcccgg aacagggggc     300

ggggccggga ctccgcgtgc ccccgccggt cgcctacgag atccactgct agggccgcgg     360

gcgcgcggat gacgtgggcg ccggagattg gccggtgcgg cctcgcgcat gctccgtcag     420

taaaggtgga agccgcgcag tgtgcgctga gccgtgtgtg gggcgcgagc cgggaacgtt     480

gcactcggga atcctggggt agcaatggaa ggtggccggg gaggccccaa cacctccctt     540

gaagctcttc cgaaaagaca tcaccccaac ctccagctct gcatgggggg ggtccctcgc     600

tggccctggc cggccacagt aggatcttgg gcctccgaaa ggtgggaaga gcaaggactg     660

gccggcagtg ccgacggggc tgccccgcga agggcggggc gacaggctgg atggcagttg     720

ttgccgggtg gccatgttgg gggtgacagc agagataagg ataaggtgga ccaacgtgga     780

cagaaaaaaa aacagccaca aattcaactt ccaggcacat gtgtaccttg atgaccccct     840

ccagcccgag cccagcccag tggggtggct gggtggcagg gtagggaaca cgtaagaggc     900

gtgagtgcct ggggactccc agcttccctg aagcaggcct ctgtccaact tcaaccccag     960

aagcactgag ctctcagtaa agggagagga aaggcccaga gaggccatca tctctctcag    1020

ctcacaactg gaatcccaaa gcatttgcac aaaggaccag gaccccagac ggcagccaaa    1080

tgcagccaaa ctgagcaggg caatggaggc ctgacatcca gccccggggc ccacagatga    1140

tcagccaatt atggagcaag tgcagggaaa accgcgtggg gcactgcaga aagcagggga    1200

ggcttgaggt gtgcccagaa gcaaggtaga cccagcagga aaccagtaac cagtgctttt    1260

gtgaaggcct ccaccagggg tcccagcaaa ggccctgcct acctaaagag actgcagggc    1320

ggcctgtgtc tcacccagag ctacttcatc ataggctgaa gtagtccgca gccagtcgcc    1380

cgtagatgtc cgtggtccag agcacatggg cacggcctga ggctagaagc agctggtgca    1440

actcagcttc tacgcgggca aacttctcct cattgatgga ggcctccagc aggacagagg    1500

caggcggcgg ccagggcaag ccctcatagc agtccacagg gaaggggtgc accacagtac    1560

gcagctcagc ctgtgccact gagtcccgca gcagctcctt gaggcccgcc atggacagtg    1620

ggttgccata gaggccaagg taccggagac tggcgcactg agtcaggatg ggtagtgtgg    1680

ccaacagctg ggtgtctgcg agctgacact cagtcagctc cagatgcaac agtgtggctg 1740

ctgatgcctg caacagaccc tggaagggtg ccagctggct gccagacagg tcgttaccac 1800

tcaggtccaa cttcttgagg tgggcagcat gtgggctccg tgccaggaag cgtaggtcct 1860

caggcagcag agcacagaag gccaactcca ggctctccag ggggctctgc agggtgctac 1920

aggggacaca ggggtcacag acaggcagag ccagggaggg tccctacaga agggaggggc 1980

agggcagtgg tggggcccgc tcacctgagc agctggtcca gcctccctga aaggagagag 2040

gagcccatgc tgagctcacg cagacaggtg aagcggccca tctgggcaag gaagtagcgg 2100

aagttgtcct cgccatccac ggagggctgc cttgaatccc catgcacata gtggagccgc 2160

aggctggcca ggtgctggaa gcgggccacg tgtgggatga tcacagacag gccgcgcagg 2220

cccagattgt tgaagcgcag gtccacgcgg cgcaggcagc ctgcatccag aagctgcagc 2280

agggccacag tgttgcgcat gggcaggtcc tcagctcgca ggtcccggca gcagagccgc 2340

agcgggctgc ccacgctgct tcggagtgcc tcccgcagga acgcatagga ggcccggttc 2400

acccgcaggt ccacgcgcac ctccacgggg atgggggctg gcccaggctc tgcggcccca 2460

ccctgctgct gggcaatgca tgtgcgagct acggcagcag tacagtccca catgctcatg 2520

gtgccaggat cctgttccac accatcatcc aagaggcccg tcatgtccag cacccgcagc 2580

gcatgcttcc tgggagtagg aatggtgtga ggaaggggtg gaggggcagc gcccataccc 2640

cctcccagcc accttgggac aggagcaacg cgcctgctcc tgcatgagct cttgctaact 2700

ttatgaccca agcctcctca ggggacccac ttacacaccc ctggcctgac gaccctccag 2760

ataagtccat acctgcagag gggctgtgtg ctggccccag gctctgaggt gtggagccgg 2820

gcagtcagcc ccaggataac agcctgcatg ctctcagtgc taggccgctc ctgcaggagg 2880

gcacggctgc agtgggcaca ctcctgtagc agctgctgga aactgagcag cgggaagggc 2940

cacgtgtgta ccaactcgcg cagtaccact gtcttcttgt ccatgaaggc caccttgaac 3000

agcaggggga agagttcgcg tggcagcaag ggcagggcct ggcaggcagc tggctggcac 3060

tgcagcacct gccgtgtgct caagaacaca agcgtgtgca tggtgctggg ccgggcagac 3120

ggccacctgc aggagaggga cttcagcagg gaggagagac cctaggccac taccaagcca 3180

cacacaagtg gtaggaaggc ccagcatagt gcctgaaaca aagtcagcag ttagcagtgg 3240

ccatggcaca gagaagcacc tattagatgc ctagcgctgg gctgggtgca gtgaactggc 3300

agacaaactc acaactcagc cccagccctc cagatgcaca ggatctcttt agagaaacta 3360

agcgtcaagc agtgtggcat cgtcaacaac tcgcttgctt gggtatgctt ttgacagatg 3420

cctgttgcct tgcagggccc cccatgggta cttcaggcct ctcaaattac aaagctgctt 3480

cacaaaacct tcttcgaaac ctgaataaag cccaggactc tccccaccaa ccttcatcag 3540

cattagtatt gacgggacac tctaaggagt caagcagcca aatcaccttt ccctcccctc 3600

tcctcacaga tgctctccag ggctacactt ggactccaca aaacaccaaa gagctggaag 3660

cacagaagca gcttcataag caacaaggca ggttgaaaag accgagaccc agggaacacc 3720

gagtccagat gatgtagcct ataaaataca agcccccgca aaagctacta ttctccagag 3780

cgtcaccaag cacggagacc ccgaaagaaa gcaaccctca cctttgttgg cccttaccac 3840

tggcctaaac ctctccctct tccctggaga gccactgtgg aaagaccaag tcttaagtat 3900

gtgggattca gaaggtgcaa agctacagac tgctcagaca aggcaaagaa agctgcctca 3960

gcagcaccca agctgcagag atgcacaaac actcccaagg ccacaaggta cctgtgacac 4020

agggtgccag ccctgctgct gcctgtagca tcctcaaaac taaatcgcag ccatctccct 4080

gtgacagatt agaaaatgaa ggcctcggcc tggcccaagg tcacagcctg agtgacaagc 4140

cagggaaggg ggccggggaa gacactctgg ccccaaggcc cttctttttg tcgacttttc 4200

cacatacaag taagtcctta aggcaccggt tcctgaaaag tcccagacac caaccctcgg 4260

cctagttctg ccggtggaca gatctcaacg atcatactcg ctctgacagg acagaccaac 4320

cgagcacttg tcacgggaga acaccaaagc agacggcctg cccaccaagg gaggcaggca 4380

cctccgtgcg acgccccctc ccctcccgcc ggccgcaggg aacgcgacgg tcctcggtcg 4440

cctgcgtttc gcgaagacgc ccccgcccgg ctcctccggg cctcgagccg cgggaggcgc 4500

tggaccctcc gctctttcgc ctcccgagcg gggcctgctc ctccaggtcg gatgcgtctc 4560

ccaccagggc ctgacgccgc tccgaccggc ccggggactc ccagtccttc ccggcccgcg 4620

gtggcacctc ccaggctccc ggcctcggcc ccgggctccc aaatgcagcc actgcctccc 4680

tcggccaggc cgccccgagc gaccggtgcc ccgccccttg aggccaggca gggccagggg 4740

cgtgcgccgc cccgctcaga cacccccccg gccgcccgcg ctcaccggtc ccgcaaccgc 4800

agccaccgcc tccagccccg cctagaccgt ccgccgctcc ccgcccggcg ccgcggcgcc 4860

ccgcgatgac gttgacgcct cccattggct gcttgtccga ggcccgacgg actggctgcc 4920

caggggcggt ggccccgccc ccggcccgcc gcgcatccgc attggctgtc ggcccccgcg 4980

acggctgcgc gggagattcg ctggacgatc gcaagcgcgg aggccgggcg ggcgcgcgcg 5040

ccatggagcg gctgcgggac gtgcgggagc ggctgcaggc gtgggagcgc gcgttccgac 5100

ggcagcgcgg gcggcgaccg agccaggtgc gggctgccca ggggccgagg ggctgagggc 5160

gcggcccgcg gctgacgcgt tccctttaca ggacgacgtg gaggcggcgc cggaggagac 5220

ccgcggtgag cgcgcggcgg ggcggcgggg gcgagaagac accgggtcgg caggggccca 5280

ggccccaccc tgaccccgcc tcccgctcgc ccacgcagcg ctctaccggg aataccgcac 5340

tctgaagcgt accacgggcc aggccggcgg cgggctccgc agctccgagt cgctccccgc 5400

ggcggccgaa gaggtaccca ggccccgccg ccccagcctc ctcccacttc cctgtttggc 5460

ggagtggcgg gagccacgga gtcgcggcca ggccgccgtg gggcacagaa cttgggaggg 5520

ggactgggca aagtgaagaa gggccgggcc tcgctccagg tgcgggaggg gtggctggga 5580

gcgcttctgc cgccacaaca gccttttctg gcctgtgccc ctgttgtctc ctgcaggcgc 5640

cagagccccg ctgctggggg ccccatctga atcgggctgc gaccaagagt ccacagccta 5700

cgccagggcg gagccgccag ggctcggtgc cggactacgg gcagcggctc aaggccaatc 5760

tgaaaggcac cctgcaggtg aggagtgggc aggcagtgag tccacgctag gtccacagct 5820

gcttccggtc cgggtcgccc tcttgtcatt ttttccacac agacaggcac gggcccctgt 5880

gccaaccagg gcacgagtct tcagggagct tctcggggcc ttcgcccttg actccctttc 5940

tagtccagcc ttgtgctaat tagcctgctc tacaattgag cgtggggact caggtaggtt 6000

ttagagtcta cagtagctca ggggcctgag ttcctcctgc tgttctgttg ttcccctccc 6060

aggccggacc agccctgggc cgcagaccgt ggcctctagg aagagcctca tctaaggcat 6120

ccaccccaaa gcccccaggt acagggcctg tcccctcctt tgcagaaaaa gtcagtgatg 6180

agcctccaca gctccctgag ccccagccaa ggccaggccg gctccagcat ctgcaggcat 6240

ccctgagcca gcggctgggc tccctagatc ctggctggtt acagcgatgt cacagtgagg 6300

tcccagattt tctgggggcc cccaaagcct gcaggcctga tctaggctca gaggaatcac 6360

aacttctgat ccctggtgag tcggctgtcc ttggtcctgg tgctggctcc cagggcccag 6420

aggcttcagc cttccaagaa gtcagcatcc gtgtggggag cccccagccc agcagcagtg 6480

gaggcgagaa gcggagatgg aacgaggagc cctgggagag ccccgcacag gtccagcagg 6540

agagcagcca agctggaccc ccatcggagg gggctggggc tgtagcagtt gaggaagacc 6600

ctccagggga acctgtacag gcacagccac ctcagccctg cagcagccca tcgaacccca 6660

ggtaccacgg actcagcccc tccagtcaag ctagggctgg gaaggctgag ggcacagccc 6720

ccctgcacat cttccctcgg ctggcccgcc atgacagggg caattacgta cggctcaaca 6780

tgaagcagaa acactacgtg cggggccggg cactccgtag caggctcctc cgcaagcagg 6840

taagacagcg acgggccagg acaggcattc cctttccctc ccctcagccc tcccgtattt 6900

cccgcccagt gaccctccta tgtgggcacc ccccaggcat ggaagcagaa gtggcggaag 6960

aaaggggagt gttttggggg tggtggtgcc acagtcacaa ccaaggagtc ttgtttcctg 7020

aacgagcagt tcgatcactg ggcagcccag tgtccccggc caggtgagac atctgccctg 7080

gagggtgggt ccggccaaca ctgtggagag ggcgcagtgc tcttttgggg gacacttatg 7140

ttccaagcaa caggccttcc aggtacccct ggtccaggcc ctaccctagc tcccctgaag 7200

gagggtggca gggacgacga tggctgtcac tcttttctgc tttggaaaaa gtagcccaga 7260

ggaagggcac tgcctgctgc caaccccctt tgggggaagg agaggttgtg gccagtggtt 7320

gtcttgcccg acctggagct cccattctac cctctcctgc ctgccccagc aagtgaggaa 7380

gacacagatg ctgttgggcc tgagccactg gttccttcac cacaacctgt acctgaggtg 7440

cccagcctgg accccaccgt gctgccactc tactccctgg ggccctcagg gcagttggca 7500

ggtgagcagt cagcttctgg cccagagcct tcactgaggg gttggggtga ctcaagtcat 7560

ggtgatcaac atctgtgtct gcagagacgc cggctgaggt gttccaggcc ctggagcagc 7620

tggggcacca agcctttcgc cctgggcagg agcgtgcagt catgcggatc ctgtctggtg 7680

agcgtggctg ccagggctga ggctgggctg aggccaggct gcagaaccct gctgctgact 7740

cccgccccat ccaggcatct ccacgctgct ggtgctgcct acaggtgccg gcaagtccct 7800

gtgctaccag ctcccagcgc tgctctacag ccggcgcagc ccctgcctca cgttggtcgt 7860

ctctcccctg ctgtcactca tggatgacca ggtgtgcaca cagggccctg ggcacacgta 7920

cacagccaag aaccagcact tgtgactccc aagggcaact gctgcttgtc ccctaaccac 7980

cccctcccct gggagcttca aggtgtctgt ggcctcagtc ccagtcttgg cagcaggtca 8040

aaggcagccc agctccacag gcaccacagc cacccctacg ggaaatgtgc tgggaaagga 8100

gccatcccta cttcagtctg tctgctctgg ggctcctggg ccaaggccca caggtggctc 8160

taaaccctta gccctaggac ccaggacctg gttctcctct cccctgaggg actaggatgg 8220

acatggcagc agctctggga tgacttgggg aagggccagg gctgggctgg cgtatgacgg 8280

ctgtcgctcc tgcatttgca ggtgtctggc ctgccaccgt gtctcaaggc ggcctgcata 8340

cactcgggca tgaccaggaa gcaacgggaa tctgtcctgc agaaggtggg ggcctcatgg 8400

gcctaggggt gagggaggca gcgggcgggc acctgggctg tgcctctgat cttgctgcct 8460

tcagattcgg gcagcccagg tacacgtgct gatgctgaca cctgaggcac tggtgggggc 8520

gggaggcctc cctccagccg cacagctgcc tccagttgct tttgcctgca ttgatgaggc 8580

ccactgcctc tcccagtggt cccacaactt ccggccctgc tacctgcgcg tctgcaaggt 8640

gagccatatg tgaactgggg tgggcggcca gggccgggat gggctgggcg gcctcacacc 8700

actgccgcct ctggtgcagg tgcttcggga gcgcatgggc gtgcactgct tcctgggcct 8760

cacagccaca gccacacgcc gcactgccag tgacgtggca cagcacctgg ctgtggctga 8820

agagcctgac ctccacgggc cagccccagt tcccaccaac ctgcaccttt ccgtgtccat 8880

ggacagggac acagaccagg tgggtgtgtg tgctctgggg accctgcagg gccctggctg 8940

ctgactgccc acgccgaccc ctcctcactc cccaccgccc acgccgaccc cttctcactc 9000

cccaccgccc acgccgaccc ctcctcactc cccacgccga cccctcctca ctccccactg 9060

cacacgccga cccctcctca ctccccactg cccacgccaa ccgctcctca tcaggcactg 9120

ttgacgctgc tgcaaggcaa acgttttcaa aacctcgatt ccattatcat ttactgcaac 9180

cggcgcgagg acacagagcg gatcgctgcg ctcctccgaa cctgcctgca cgcagcctgg 9240

gtcccagggt ctggaggtgc ggcatggaca gagctggtgt ccccgtggac ccaccttggg 9300

cacacatggt cccatcccac tgaccatctg cctgtcttcc ccaaaggtcg tgcccccaaa 9360

accacagccg aggcctacca cgcgggcatg tgcagccggg aacggcggcg ggtacagcga 9420

gccttcatgc agggccagtt gcgggtggtg gtggccacgg tggcctttgg gatggggctg 9480

gaccggccag atgtgcgggc tgtgctgcat ctggggctgc ccccaagctt cgagagctac 9540

gtgcaggccg tgggccgggc cgggcgtgac gggcagcctg cccactgcca cctcttcctg 9600

cagccccagg ttggcacccc ccccccccac actgccagtg ctcgagcccc cagtggtcca 9660

ccccaccctc atgaaagttg ccctgcaggg cgaagacctg cgagagctgc gcagacatgt 9720

gcacgccgac agcacggact tcctggctgt gaagaggctg gtacagcgcg tgttcccagc 9780

ctgcacctgc acctgcacca ggccgccctc ggagcaggaa ggggccgtgg gtggggagag 9840

gcctgtgccc aagtaccccc ctcaagaggc tgagcagctt agccaccaag cagccccagg 9900

acccagaagg gtctgcatgg gccatgagcg ggcactccca atacagctta ccgtacaggc 9960

tttggacatg ccggaggagg gtgaggaacc tggggtaagc cacaggggtg tggaggggct 10020

gtccccgcgt ccgctgagcc ctgctctgcc cccagccatc gagactttgc tgtgctacct 10080

ggagctgcac ccacaccact ggctggagct gctggcgacc acctataccc attgccgtct 10140

gaactgccct gggggccctg cccagctcca ggccctggcc cacaggtaag cacgccctgc 10200

ccagttggag acgaggttgg agaatcaggg ctgttggcca catgtccctt tttccctggg 10260

cacaggtgtc cccctttggc tgtgtgcttg gcccagcagc tgcctgagga cccagggcaa 10320

ggcagcagct ccgtggagtt tgacatggtc aagctggtgg actccatggg ctgggagctg 10380

gcctctgtgc ggcgggctct ctgccagctg cagtgggacc acgagcccag gacaggtgcg 10440

cctctcccca ccccacaccg ccctggacgc tgcctgcctg catctgacat gctttccggc 10500

aggtgtgcgg cgtgggacag gggtgcttgt ggagttcagt gagctggcct tccaccttcg 10560

cagcccgggg gacctgaccg ctgaggagaa ggaccagata tgtgacttcc tctatggccg 10620

tgtgcaggcc cgggagcgcc aggccctggc ccgtctgcgc agaaccttcc aggcctttca 10680

caggttggga ggaggcgggc ggggcctggg accatccacc ctcccgcagt gatcagctct 10740

gacaggctcc tccccacagc gtagccttcc ccagctgcgg gccctgcctg gagcagcagg 10800

atgaggagcg cagcaccagg ctcaaggacc tgctcggccg ctactttgag gaagaggaag 10860

ggcaggagcc gggaggcatg gaggacgcac agggccccga gccagggcag gccagagtga 10920

gtgcagtaag gccaggcagc tcatcggggt tgcaggttcc ctgggctgca tggggcttgc 10980

tctgtggatg cagtgccacg ggagctcaga ggaagcctga tgtgcctgtc cacacagctc 11040

caggattggg aggaccaggt ccgctgcgac atccgccagt tcctgtccct gaggccagag 11100

gagaagttct ccagcagggc tgtggcccgc atcttccacg gcatcggtga ggcctgggag 11160

gccccacccg ctgcaggctg gggctggggg ctggggcagg tgaggcctgg gaggctccac 11220

ccgctgcagg ctggggctgg ggctcacggc tgtgtcttgg ctccaccgta ggaagcccct 11280

gctacccggc ccaggtgtac gggcaggacc gacgcttctg gagaaaatac ctgcacctga 11340

gcttccatgc cctggtgggc ctggccacgg aagagctcct gcaggtggcc cgctgactgc 11400

actgcattgg gggatgtcgg gtagagctgg ggttgtcaga ggctagggca gtgactgagg 11460

acctgggcaa aacctgccac agggtgtggg aacgaggagg ctccaaaatg cagaataaaa 11520

aatgctcact ttgtttttat gggccctctt ggccaatgag gcaaccccca caagctgggg 11580

acaagcacat cacatattcc aggcacagag acatctgggc cacaggcaca tgtggcttta 11640

ttgaccactc cagccactca gctcagagaa aggtgctggg tgctaggtcc aggtacagaa 11700

cgggaaaggc agagaggatg agcaggagca agaagcaggg atgtggcccc aacccatcag 11760

ccaggcctcc ggccagagtg cccagcagca gcttccccag cagctccagc gtggccagaa 11820

ggctgtagtg tgtggcctga gggggtgggg ggtcacggtc aaggtcaggc ccacacagcc 11880

ccagccccgt ggtctctctt gtgtccctgc tacatctact cacctgcagg gccctggggg 11940

ccagctggct gcagcgcatc atcccagtga aggtgactgt ggtgaccagg cctcccaaga 12000

agtgctgcag acataggctc agcaaggctg accctgggag ggcagaagtc agcagtgccc 12060

caggctcctg gcccctctct tcctcctcaa ggccagcccc tcacctctca agattgtgcc 12120

agcgtccatg ctggccccca gggtgtccag gtggaagacc aaggcagtct gacaggctag 12180

gcccccgagg cggaagcgca gcaccgacct caacagaggc agcagtttcc tgggatgggg 12240

gtgggaaatg ggtcagtata gtggattggg ggcagctcag gtacaagtgg gtgggcaggg 12300

tacactgggg agggctcacc agtgcttggc cagcaaggtc ccacccaggg aggagccagc 12360

gatggagcag accacagcac ccacaccatt ccacagtccc aactcgggag cagaaacgcc 12420

gtggtccagc aggagaagag gaaacaggct gctggcaccc tgctcacctt gggtggtggg 12480

gggggcatga gcacgggagg ttccatatct gcccccacct cccttctccc cacctgggcc 12540

ctcagtcctg ccctgctctc ctcagtgttc cagagctgca gatactgggg gagaagtggg 12600

gcagcccgag agagcgctca gggatggggc tagagctggg ggcggacaag acacaaggcc 12660

cagctgcaga gccccggatg tcagggccaa gtcttgcagg tcatacccca gaaagagcgt 12720

ggccctgtaa tgggttgcaa gcccctgggg acacacctgg agctgtcccg gaagccccaa 12780

ctgctgttgc ctggctcgag gcctcactca cccagcttgt aggtgagcac aaagcctgcc 12840

gtccacacgg tccccggcac ggctagcacg tcccgcagaa ggtgcgcggt gtggggacgc 12900

tgctcggaag ggggctgctg tgggagccgc cgcagggctg gtgcagccca ggccagggcc 12960

gcggccagcc agtaggtggc agccaggagc agaaagagtt gcggccacga gaaggtgggc 13020

agcagcgcca gcagcgcgcc cccagctagc gcggccccca gcttgtacgc gaccacctgc 13080

acggtattgc ccggccccag ttcggccggc tccagcagct gcacagccag cgcgtccagg 13140

gccacatcct gcatggcggc acccaggttc aacaacagca gcaaccccgc cacagcggcg 13200

ggcagcccgg cctggccagc tccaggaggg ggcagcccgg caagcagccc acacaccagg 13260

cccaggcccg ccgtgctgcg cgtcacccag gccctcgccg agccctgcgc gtccaccagc 13320

ggggcccaag ccagcttgag cagccacgga gcgtacagaa ccttggccag ccccacgcgc 13380

gtcagcgaga ggccgccggc acgcagcagc actggcagga ggccggactg gagcccgtag 13440

ggcaggccct gcaccaggta gaggccggcc agcggcagca acttcccgcg catggcggct 13500

caggccggct gcgaggctgg gaggtcgggt tccaggtggg gtc 13543

<210> 5
<211> 13543
<212> DNA
<213> Homo sapiens

<400> 5
   cgccctgggc gtggccacac agacggccat tgcggcggcc atcgcgctgc tggcgctcac 60
   ggcgctgctc ctggtcgcca tgatctgtcg ccggcgccgc aggcgaaaaa aggcgcgggg 120
   gcctccgggg gagggagcgc tgttcgtcaa cgactacttg gacggcccct gtacgttcgc 180
   acagctagag gagctccgcg acgagcgcgg ccacgagatg ttcgtcatca accgctccaa 240
   gccgctcttc gccgagggtc cggcggaggc gcccgctgac tgcggcccgg aacagggggc 300
   ggggccggga ctccgcgtgc ccccgccggt cgcctacgag atccactgct agggccgcgg 360
   gcgcgcggat gacgtgggcg ccggagattg gccggtgcgg cctcgcgcat gctccgtcag 420
   taaaggtgga agccgcgcag tgtgcgctga gccgtgtgtg gggcgcgagc cgggaacgtt 480
   gcactcggga atcctggggt agcaatggaa ggtggccggg gaggccccaa cacctccctt 540
   gaagctcttc cgaaaagaca tcaccccaac ctccagctct gcatgggggg ggtccctcgc 600
   tggccctggc cggccacagt aggatcttgg gcctccgaaa ggtgggaaga gcaaggactg 660
   gccggcagtg ccgacggggc tgccccgcga agggcggggc gacaggctgg atggcagttg 720
   ttgccgggtg gccatgttgg gggtgacagc agagataagg ataagggtga ccaacgtgga 780
   cagaaaaaaa aacagccaca aattcaactt ccaggcacat gtgtaccttg atgaccccct 840
   ccagcccgag cccagcccag tggggtggct gggtggcagg gtagggaaca cgtaagaggc 900
   gtgagtgcct ggggactccc agcttccctg aagcaggcct ctgtccaact tcaaccccag 960
   aagcactgag ctctcagtaa agggagagga aaggcccaga gaggccatca tctctctcag 1020
   ctcacaactg gaatcccaaa gcatttgcac aaaggaccag gaccccagac ggcagccaaa 1080
   tgcagccaaa ctgagcaggg caatggaggc ctgacatcca gccccggggc ccacagatga 1140
   tcagccaatt atggagcaag tgcagggaaa accgcgtggg gcactgcaga aagcagggga 1200
   ggcttgaggt gtgcccagaa gcaaggtaga cccagcagga aaccagtaac cagtgctttt 1260
   gtgaaggcct ccaccagggg tcccagcaaa ggccctgcct acctaaagag actgcagggc 1320
   ggcctgtgtc tcacccagag ctacttcatc ataggctgaa gtagtccgca gccagtcgcc 1380
   cgtagatgtc cgtggtccag agcacatggg cacggcctga ggctagaagc agctggtgca 1440
   actcagcttc tacgcgggca aacttctcct cattgatgga ggcctccagc aggacagagg 1500
   caggcggcgg ccagggcaag ccctcatagc agtccacagg gaaggggtgc accacagtac 1560
   gcagctcagc ctgtgccact gagtcccgca gcagctcctt gaggcccgcc atggacagtg 1620
   ggttgccata gaggccaagg taccggagac tggcgcactg agtcaggatg ggtagtgtgg 1680
   ccaacagctg ggtgtctgcg agctgacact cagtcagctc cagatgcaac agtgtggctg 1740
   ctgatgcctg caacagaccc tggaagggtg ccagctggct gccagacagg tcgttaccac 1800
   tcaggtccaa cttcttgagg tgggcagcat gtgggctccg tgccaggaag cgtaggtcct 1860
   caggcagcag agcacagaag gccaactcca ggctctccag ggggctctgc agggtgctac 1920
   aggggacaca ggggtcacag acaggcagag ccagggaggg tccctacaga agggaggggc 1980
   agggcagtgg tggggcccgc tcacctgagc agctggtcca gcctccctga aaggagagag 2040
   gagcccatgc tgagctcacg cagacaggtg aagcggccca tctgggcaag gaagtagcgg 2100
   aagttgtcct cgccatccac ggagggctgc cttgaatccc catgcacata gtggagccgc 2160
   aggctggcca ggtgctggaa gcgggccacg tgtgggatga tcacagacag gccgcgcagg 2220
   cccagattgt tgaagcgcag gtccacgcgg cgcaggcagc ctgcatccag aagctgcagc 2280
   agggccacag tgttgcgcat gggcaggtcc tcagctcgca ggtcccggca gcagagccgc 2340
   agcgggctgc ccacgctgct tcggagtgcc tcccgcagga acgcatagga ggcccggttc 2400
   acccgcaggt ccacgcgcac ctccacgggg atgggggctg gcccaggctc tgcggcccca 2460
   ccctgctgct gggcaatgca tgtgcgagct acggcagcag tacagtccca catgctcatg 2520
   gtgccaggat cctgttccac accatcatcc aagaggcccg tcatgtccag cacccgcagc 2580
   gcatgcttcc tgggagtagg aatggtgtga ggaaggggtg gaggggcagc gcccataccc 2640
   cctcccagcc accttgggac aggagcaacg cgcctgctcc tgcatgagct cttgctaact 2700
   ttatgaccca agcctcctca ggggacccac ttacacaccc ctggcctgac gaccctccag 2760
   ataagtccat acctgcagag gggctgtgtg ctggccccag gctctgaggt gtggagccgg 2820
   gcagtcagcc ccaggataac agcctgcatg ctctcagtgc taggccgctc ctgcaggagg 2880
   gcacggctgc agtgggcaca ctcctgtagc agctgctgga aactgagcag cgggaagggc 2940
   cacgtgtgta ccaactcgcg cagtaccact gtcttcttgt ccatgaaggc caccttgaac 3000
   agcaggggga agagttcgcg tggcagcaag ggcagggcct ggcaggcagc tggctggcac 3060
   tgcagcacct gccgtgtgct caagaacaca agcgtgtgca tggtgctggg ccgggcagac 3120
   ggccacctgc aggagaggga cttcagcagg gaggagagac cctaggccac taccaagcca 3180
   cacacaagtg gtaggaaggc ccagcatagt gcctgaaaca aagtcagcag ttagcagtgg 3240
   ccatggcaca gagaagcacc tattagatgc ctagcgctgg gctgggtgca gtgaactggc 3300
   agacaaactc acaactcagc cccagccctc cagatgcaca ggatctcttt agagaaacta 3360
   agcgtcaagc agtgtggcat cgtcaacaac tcgcttgctt gggtatgctt ttgacagatg 3420
   cctgttgcct tgcagggccc cccatgggta cttcaggcct ctcaaattac aaagctgctt 3480
   cacaaaacct tcttcgaaac ctgaataaag cccaggactc tccccaccaa ccttcatcag 3540
   cattagtatt gacgggacac tctaaggagt caagcagcca aatcaccttt ccctcccctc 3600
   tcctcacaga tgctctccag ggctacactt ggactccaca aaacaccaaa gagctggaag 3660
   cacagaagca gcttcataag caacaaggca ggttgaaaag accgagaccc agggaacacc 3720
   gagtccagat gatgtagcct ataaaataca agcccccgca aaagctacta ttctccagag 3780
   cgtcaccaag cacggagacc ccgaaagaaa gcaaccctca cctttgttgg cccttaccac 3840
   tggcctaaac ctctccctct tccctggaga gccactgtgg aaagaccaag tcttaagtat 3900
   gtgggattca gaaggtgcaa agctacagac tgctcagaca aggcaaagaa agctgcctca 3960
   gcagcaccca agctgcagag atgcacaaac actcccaagg ccacaaggta cctgtgacac 4020
   agggtgccag ccctgctgct gcctgtagca tcctcaaaac taaatcgcag ccatctccct 4080
   gtgacagatt agaaaatgaa ggcctcggcc tggcccaagg tcacagcctg agtgacaagc 4140
   cagggaaggg ggccggggaa gacactctgg ccccaaggcc cttctttttg tcgacttttc 4200
   cacatacaag taagtcctta aggcaccggt tcctgaaaag tcccagacac caaccctcgg 4260
   cctagttctg ccggtggaca gatctcaacg atcatactcg ctctgacagg acagaccaac 4320
   cgagcacttg tcacgggaga acaccaaagc agacggcctg cccaccaagg gaggcaggca 4380
   cctccgtgcg acgccccctc ccctcccgcc ggccgcaggg aacgcgacgg tcctcggtcg 4440
   cctgcgtttc gcgaagacgc ccccgcccgg ctcctccggg cctcgagccg cgggaggcgc 4500
   tggaccctcc gctctttcgc ctcccgagcg gggcctgctc ctccaggtcg gatgcgtctc 4560
   ccaccagggc ctgacgccgc tccgaccggc ccggggactc ccagtccttc ccggcccgcg 4620
   gtggcacctc ccaggctccc ggcctcggcc ccgggctccc aaatgcagcc actgcctccc 4680
   tcggccaggc cgccccgagc gaccggtgcc ccgccccttg aggccaggca gggccagggg 4740
   cgtgcgccgc cccgctcaga cacccccccg gccgcccgcg ctcaccggtc ccgcaaccgc 4800
   agccaccgcc tccagccccg cctagaccgt ccgccgctcc ccgcccggcg ccgcggcgcc 4860
   ccgcgatgac gttgacgcct cccattggct gcttgtccga ggcccgacgg actggctgcc 4920
   caggggcggt ggccccgccc ccggcccgcc gcgcatccgc attggctgtc ggcccccgcg 4980
   acggctgcgc gggagattcg ctggacgatc gcaagcgcgg aggccgggcg ggcgcgcgcg 5040
   ccatggagcg gctgcgggac gtgcgggagc ggctgcaggc gtgggagcgc gcgttccgac 5100
   ggcagcgcgg gcggcgaccg agccaggtgc gggctgccca ggggccgagg ggctgagggc 5160
   gcggcccgcg gctgacgcgt tccctttaca ggacgacgtg gaggcggcgc cggaggagac 5220
   ccgcggtgag cgcgcggcgg ggcggcgggg gcgagaagac accgggtcgg caggggccca 5280
   ggccccaccc tgaccccgcc tcccgctcgc ccacgcagcg ctctaccggg aataccgcac 5340
   tctgaagcgt accacgggcc aggccggcgg cgggctccgc agctccgagt cgctccccgc 5400
   ggcggccgaa gaggtaccca ggccccgccg ccccagcctc ctcccacttc cctgtttggc 5460
   ggagtggcgg gagccacgga gtcgcggcca ggccgccgtg gggcacagaa cttgggaggg 5520
   ggactgggca aagtgaagaa gggccgggcc tcgctccagg tgcgggaggg gtggctggga 5580
   gcgcttctgc cgccacaaca gccttttctg gcctgtgccc ctgttgtctc ctgcaggcgc 5640
   cagagccccg ctgctggggg ccccatctga atcgggctgc gaccaagagt ccacagccta 5700
   cgccagggcg gagccgccag ggctcggtgc cggactacgg gcagcggctc aaggccaatc 5760
   tgaaaggcac cctgcaggtg aggagtgggc aggcagtgag tccacgctag gtccacagct 5820
   gcttccggtc cgggtcgccc tcttgtcatt ttttccacac agacaggcac gggcccctgt 5880
   gccaaccagg gcacgagtct tcagggagct tctcggggcc ttcgcccttg actccctttc 5940
   tagtccagcc ttgtgctaat tagcctgctc tacaattgag cgtggggact caggtaggtt 6000
   ttagagtcta cagtagctca ggggcctgag ttcctcctgc tgttctgttg ttcccctccc 6060
   aggccggacc agccctgggc cgcagaccgt ggcctctagg aagagcctca tctaaggcat 6120
   ccaccccaaa gcccccaggt acagggcctg tcccctcctt tgcagaaaaa gtcagtgatg 6180
   agcctccaca gctccctgag ccccagccaa ggccaggccg gctccagcat ctgcaggcat 6240
   ccctgagcca gcggctgggc tccctagatc ctggctggtt acagcgatgt cacagtgagg 6300
   tcccagattt tctgggggcc cccaaagcct gcaggcctga tctaggctca gaggaatcac 6360
   aacttctgat ccctggtgag tcggctgtcc ttggtcctgg tgctggctcc cagggcccag 6420
   aggcttcagc cttccaagaa gtcagcatcc gtgtggggag cccccagccc agcagcagtg 6480
   gaggcgagaa gcggagatgg aacgaggagc cctgggagag ccccgcacag gtccagcagg 6540
   agagcagcca agctggaccc ccatcggagg gggctggggc tgtagcagtt gaggaagacc 6600
   ctccagggga acctgtacag gcacagccac ctcagccctg cagcagccca tcgaacccca 6660
   ggtaccacgg actcagcccc tccagtcaag ctagggctgg gaaggctgag ggcacagccc 6720
   ccctgcacat cttccctcgg ctggcccgcc atgacagggg caattacgta cggctcaaca 6780
   tgaagcagaa acactacgtg cggggccggg cactccgtag caggctcctc cgcaagcagg 6840
   taagacagcg acgggccagg acaggcattc cctttccctc ccctcagccc tcccgtattt 6900
   cccgcccagt gaccctccta tgtgggcacc ccccaggcat ggaagcagaa gtggcggaag 6960
   aaaggggagt gttttggggg tggtggtgcc acagtcacaa ccaaggagtc ttgtttcctg 7020
   aacgagcagt tcgatcactg ggcagcccag tgtccccggc caggtgagac atctgccctg 7080
   gagggtgggt ccggccaaca ctgtggagag ggcgcagtgc tcttttgggg gacacttatg 7140
   ttccaagcaa caggccttcc aggtacccct ggtccaggcc ctaccctagc tcccctgaag 7200
   gagggtggca gggacgacga tggctgtcac tcttttctgc tttggaaaaa gtagcccaga 7260
   ggaagggcac tgcctgctgc caaccccctt tgggggaagg agaggttgtg gccagtggtt 7320
   gtcttgcccg acctggagct cccattctac cctctcctgc ctgccccagc aagtgaggaa 7380
   gacacagatg ctgttgggcc tgagccactg gttccttcac cacaacctgt acctgaggtg 7440
   cccagcctgg accccaccgt gctgccactc tactccctgg ggccctcagg gcagttggca 7500
   ggtgagcagt cagcttctgg cccagagcct tcactgaggg gttggggtga ctcaagtcat 7560
   ggtgatcaac atctgtgtct gcagagacgc cggctgaggt gttccaggcc ctggagcagc 7620
   tggggcacca agcctttcgc cctgggcagg agcgtgcagt catgcggatc ctgtctggtg 7680
   agcgtggctg ccagggctga ggctgggctg aggccaggct gcagaaccct gctgctgact 7740
   cccgccccat ccaggcatct ccacgctgct ggtgctgcct acaggtgccg gcaagtccct 7800
   gtgctaccag ctcccagcgc tgctctacag ccggcgcagc ccctgcctca cgttggtcgt 7860
   ctcttccctg ctgtcactca tggatgacca ggtgtgcaca cagggccctg ggcacacgta 7920
   cacagccaag aaccagcact tgtgactccc aagggcaact gctgcttgtc ccctaaccac 7980
   cccctcccct gggagcttca aggtgtctgt ggcctcagtc ccagtcttgg cagcaggtca 8040
   aaggcagccc agctccacag gcaccacagc cacccctacg ggaaatgtgc tgggaaagga 8100
   gccatcccta cttcagtctg tctgctctgg ggctcctggg ccaaggccca caggtggctc 8160
   taaaccctta gccctaggac ccaggacctg gttctcctct cccctgaggg actaggatgg 8220
   acatggcagc agctctggga tgacttgggg aagggccagg gctgggctgg cgtatgacgg 8280
   ctgtcgctcc tgcatttgca ggtgtctggc ctgccaccgt gtctcaaggc ggcctgcata 8340
   cactcgggca tgaccaggaa gcaacgggaa tctgtcctgc agaaggtggg ggcctcatgg 8400
   gcctaggggt gagggaggca gcgggcgggc acctgggctg tgcctctgat cttgctgcct 8460
   tcagattcgg gcagcccagg tacacgtgct gatgctgaca cctgaggcac tggtgggggc 8520
   gggaggcctc cctccagccg cacagctgcc tccagttgct tttgcctgca ttgatgaggc 8580
   ccactgcctc tcccagtggt cccacaactt ccggccctgc tacctgcgcg tctgcaaggt 8640
   gagccatatg tgaactgggg tgggcggcca gggccgggat gggctgggcg gcctcacacc 8700
   actgccgcct ctggtgcagg tgcttcggga gcgcatgggc gtgcactgct tcctgggcct 8760
   cacagccaca gccacacgcc gcactgccag tgacgtggca cagcacctgg ctgtggctga 8820
   agagcctgac ctccacgggc cagccccagt tcccaccaac ctgcaccttt ccgtgtccat 8880
   ggacagggac acagaccagg tgggtgtgtg tgctctgggg accctgcagg gccctggctg 8940
   ctgactgccc acgccgaccc ctcctcactc cccaccgccc acgccgaccc cttctcactc 9000
   cccaccgccc acgccgaccc ctcctcactc cccacgccga cccctcctca ctccccactg 9060
   cacacgccga cccctcctca ctccccactg cccacgccaa ccgctcctca tcaggcactg 9120
   ttgacgctgc tgcaaggcaa acgttttcaa aacctcgatt ccattatcat ttactgcaac 9180
   cggcgcgagg acacagagcg gatcgctgcg ctcctccgaa cctgcctgca cgcagcctgg 9240
   gtcccagggt ctggaggtgc ggcatggaca gagctggtgt ccccgtggac ccaccttggg 9300
   cacacatggt cccatcccac tgaccatctg cctgtcttcc ccaaaggtcg tgcccccaaa 9360
   accacagccg aggcctacca cgcgggcatg tgcagccggg aacggcggcg ggtacagcga 9420
   gccttcatgc agggccagtt gcgggtggtg gtggccacgg tggcctttgg gatggggctg 9480
   gaccggccag atgtgcgggc tgtgctgcat ctggggctgc ccccaagctt cgagagctac 9540
   gtgcaggccg tgggccgggc cgggcgtgac gggcagcctg cccactgcca cctcttcctg 9600
   cagccccagg ttggcacccc ccccccccac actgccagtg ctcgagcccc cagtggtcca 9660
   ccccaccctc atgaaagttg ccctgcaggg cgaagacctg cgagagctgc gcagacatgt 9720
   gcacgccgac agcacggact tcctggctgt gaagaggctg gtacagcgcg tgttcccagc 9780
   ctgcacctgc acctgcacca ggccgccctc ggagcaggaa ggggccgtgg gtggggagag 9840
   gcctgtgccc aagtaccccc ctcaagaggc tgagcagctt agccaccaag cagccccagg 9900
   acccagaagg gtctgcatgg gccatgagcg ggcactccca atacagctta ccgtacaggc 9960
   tttggacatg ccggaggagg gtgaggaacc tggggtaagc cacaggggtg tggaggggct 10020
   gtccccgcgt ccgctgagcc ctgctctgcc cccagccatc gagactttgc tgtgctacct 10080
   ggagctgcac ccacaccact ggctggagct gctggcgacc acctataccc attgccgtct 10140
   gaactgccct gggggccctg cccagctcca ggccctggcc cacaggtaag cacgccctgc 10200
   ccagttggag acgaggttgg agaatcaggg ctgttggcca catgtccctt tttccctggg 10260
   cacaggtgtc cccctttggc tgtgtgcttg gcccagcagc tgcctgagga cccagggcaa 10320
   ggcagcagct ccgtggagtt tgacatggtc aagctggtgg actccatggg ctgggagctg 10380
   gcctctgtgc ggcgggctct ctgccagctg cagtgggacc acgagcccag gacaggtgcg 10440
   cctctcccca ccccacaccg ccctggacgc tgcctgcctg catctgacat gctttccggc 10500
   aggtgtgcgg cgtgggacag gggtgcttgt ggagttcagt gagctggcct tccaccttcg 10560
   cagcccgggg gacctgaccg ctgaggagaa ggaccagata tgtgacttcc tctatggccg 10620
   tgtgcaggcc cgggagcgcc aggccctggc ccgtctgcgc agaaccttcc aggcctttca 10680
   caggttggga ggaggcgggc ggggcctggg accatccacc ctcccgcagt gatcagctct 10740
   gacaggctcc tccccacagc gtagccttcc ccagctgcgg gccctgcctg gagcagcagg 10800
   atgaggagcg cagcaccagg ctcaaggacc tgctcggccg ctactttgag gaagaggaag 10860
   ggcaggagcc gggaggcatg gaggacgcac agggccccga gccagggcag gccagagtga 10920
   gtgcagtaag gccaggcagc tcatcggggt tgcaggttcc ctgggctgca tggggcttgc 10980
   tctgtggatg cagtgccacg ggagctcaga ggaagcctga tgtgcctgtc cacacagctc 11040
   caggattggg aggaccaggt ccgctgcgac atccgccagt tcctgtccct gaggccagag 11100
   gagaagttct ccagcagggc tgtggcccgc atcttccacg gcatcggtga ggcctgggag 11160
   gccccacccg ctgcaggctg ggctgggggg ctggggcagg tgaggcctgg gaggctccac 11220
   ccgctgcagg ctggggctgg ggctcacggc tgtgtcttgg ctccaccgta ggaagcccct 11280
   gctacccggc ccaggtgtac gggcaggacc gacgcttctg gagaaaatac ctgcacctga 11340
   gcttccatgc cctggtgggc ctggccacgg aagagctcct gcaggtggcc cgctgactgc 11400
   actgcattgg gggatgtcgg gtagagctgg ggttgtcaga ggctagggca gtgactgagg 11460
   acctgggcaa aacctgccac agggtgtggg aacgaggagg ctccaaaatg cagaataaaa 11520
   aatgctcact ttgtttttat gggccctctt ggccaatgag gcaaccccca caagctgggg 11580
   acaagcacat cacatattcc aggcacagag acatctgggc cacaggcaca tgtggcttta 11640
   ttgaccactc cagccactca gctcagagaa aggtgctggg tgctaggtcc aggtacagaa 11700
   cgggaaaggc agagaggatg agcaggagca agaagcaggg atgtggcccc aacccatcag 11760
   ccaggcctcc ggccagagtg cccagcagca gcttccccag cagctccagc gtggccagaa 11820
   ggctgtagtg tgtggcctga ggggggtggg ggtcacggtc aaggtcaggc ccacacagcc 11880
   ccagccccgt ggtctctctt gtgtccctgc tacatctact cacctgcagg gccctggggg 11940
   ccagctggct gcagcgcatc atcccagtga aggtgactgt ggtgaccagg cctcccaaga 12000
   agtgctgcag acataggctc agcaaggctg accctgggag ggcagaagtc agcagtgccc 12060
   caggctcctg gcccctctct tcctcctcaa ggccagcccc tcacctctca agattgtgcc 12120
   agcgtccatg ctggccccca gggtgtccag gtggaagacc aaggcagtct gacaggctag 12180
   gcccccgagg cggaagcgca gcaccgacct caacagaggc agcagtttcc tgggatgggg 12240
   gtgggaaatg ggtcagtata gtggattggg ggcagctcag gtacaagtgg gtgggcaggg 12300
   tacactgggg agggctcacc agtgcttggc cagcaaggtc ccacccaggg aggagccagc 12360
   gatggagcag accacagcac ccacaccatt ccacagtccc aactcgggag cagaaacgcc 12420
   gtggtccagc aggagaagag gaaacaggct gctggcaccc tgctcacctt gggtggtggg 12480
   gggggcatga gcacgggagg ttccatatct gcccccacct cccttctccc cacctgggcc 12540
   ctcagtcctg ccctgctctc ctcagtgttc cagagctgca gatactgggg gagaagtggg 12600
   gcagcccgag agagcgctca gggatggggc tagagctggg ggcggacaag acacaaggcc 12660
   cagctgcaga gccccggatg tcagggccaa gtcttgcagg tcatacccca gaaagagcgt 12720
   ggccctgtaa tgggttgcaa gcccctgggg acacacctgg agctgtcccg gaagccccaa 12780
   ctgctgttgc ctggctcgag gcctcactca cccagcttgt aggtgagcac aaagcctgcc 12840
   gtccacacgg tccccggcac ggctagcacg tcccgcagaa ggtgcgcggt gtggggacgc 12900
   tgctcggaag ggggctgctg tgggagccgc cgcagggctg gtgcagccca ggccagggcc 12960
   gcggccagcc agtaggtggc agccaggagc agaaagagtt gcggccacga gaaggtgggc 13020
   agcagcgcca gcagcgcgcc cccagctagc gcggccccca gcttgtacgc gaccacctgc 13080
   acggtattgc ccggccccag ttcggccggc tccagcagct gcacagccag cgcgtccagg 13140
   gccacatcct gcatggcggc acccaggttc aacaacagca gcaaccccgc cacagcggcg 13200
   ggcagcccgg cctggccagc tccaggaggg ggcagcccgg caagcagccc acacaccagg 13260
   cccaggcccg ccgtgctgcg cgtcacccag gccctcgccg agccctgcgc gtccaccagc 13320
   ggggcccaag ccagcttgag cagccacgga gcgtacagaa ccttggccag ccccacgcgc 13380
   gtcagcgaga ggccgccggc acgcagcagc actggcagga ggccggactg gagcccgtag 13440
   ggcaggccct gcaccaggta gaggccggcc agcggcagca acttcccgcg catggcggct 13500
   caggccggct gcgaggctgg gaggtcgggt tccaggtggg gtc 13543

<210> 6
<211> 13543
<212> DNA
<213> Homo sapiens

<400> 6
cgccctgggc gtggccacac agacggccat tgcggcggcc atcgcgctgc tggcgctcac 60

ggcgctgctc ctggtcgcca tgatctgtcg ccggcgccgc aggcgaaaaa aggcgcgggg 120

gcctccgggg gagggagcgc tgttcgtcaa cgactacttg gacggcccct gtacgttcgc 180

acagctagag gagctccgcg acgagcgcgg ccacgagatg ttcgtcatca accgctccaa 240

gccgctcttc gccgagggtc cggcggaggc gcccgctgac tgcggcccgg aacagggggc 300

ggggccggga ctccgcgtgc ccccgccggt cgcctacgag atccactgct agggccgcgg 360

gcgcgcggat gacgtgggcg ccggagattg gccggtgcgg cctcgcgcat gctccgtcag 420

taaaggtgga agccgcgcag tgtgcgctga gccgtgtgtg gggcgcgagc cgggaacgtt 480

gcactcggga atcctggggt agcaatggaa ggtggccggg gaggccccaa cacctccctt 540

gaagctcttc cgaaaagaca tcaccccaac ctccagctct gcatgggggg ggtccctcgc 600

tggccctggc cggccacagt aggatcttgg gcctccgaaa ggtgggaaga gcaaggactg 660

gccggcagtg ccgacggggc tgccccgcga agggcggggc gacaggctgg atggcagttg 720

ttgccgggtg gccatgttgg gggtgacagc agagataagg ataagggtga ccaacgtgga 780

cagaaaaaaa aacagccaca aattcaactt ccaggcacat gtgtaccttg atgaccccct 840

ccagcccgag cccagcccag tggggtggct gggtggcagg gtagggaaca cgtaagaggc 900

gtgagtgcct ggggactccc agcttccctg aagcaggcct ctgtccaact tcaaccccag 960

aagcactgag ctctcagtaa agggagagga aaggcccaga gaggccatca tctctctcag 1020

ctcacaactg gaatcccaaa gcatttgcac aaaggaccag gaccccagac ggcagccaaa 1080

tgcagccaaa ctgagcaggg caatggaggc ctgacatcca gccccggggc ccacagatga 1140

tcagccaatt atggagcaag tgcagggaaa accgcgtggg gcactgcaga aagcagggga 1200

ggcttgaggt gtgcccagaa gcaaggtaga cccagcagga aaccagtaac cagtgctttt 1260

gtgaaggcct ccaccagggg tcccagcaaa ggccctgcct acctaaagag actgcagggc 1320

ggcctgtgtc tcacccagag ctacttcatc ataggctgaa gtagtccgca gccagtcgcc 1380

cgtagatgtc cgtggtccag agcacatggg cacggcctga ggctagaagc agctggtgca 1440

actcagcttc tacgcgggca aacttctcct cattgatgga ggcctccagc aggacagagg 1500

caggcggcgg ccagggcaag ccctcatagc agtccacagg gaaggggtgc accacagtac 1560

gcagctcagc ctgtgccact gagtcccgca gcagctcctt gaggcccgcc atggacagtg 1620

ggttgccata gaggccaagg taccggagac tggcgcactg agtcaggatg ggtagtgtgg 1680

ccaacagctg ggtgtctgcg agctgacact cagtcagctc cagatgcaac agtgtggctg 1740

ctgatgcctg caacagaccc tggaagggtg ccagctggct gccagacagg tcgttaccac 1800

tcaggtccaa cttcttgagg tgggcagcat gtgggctccg tgccaggaag cgtaggtcct 1860

caggcagcag agcacagaag gccaactcca ggctctccag ggggctctgc agggtgctac 1920

aggggacaca ggggtcacag acaggcagag ccagggaggg tccctacaga agggaggggc 1980

agggcagtgg tggggcccgc tcacctgagc agctggtcca gcctccctga aaggagagag 2040

gagcccatgc tgagctcacg cagacaggtg aagcggccca tctgggcaag gaagtagcgg 2100

aagttgtcct cgccatccac ggagggctgc cttgaatccc catgcacata gtggagccgc 2160

aggctggcca ggtgctggaa gcgggccacg tgtgggatga tcacagacag gccgcgcagg 2220

cccagattgt tgaagcgcag gtccacgcgg cgcaggcagc ctgcatccag aagctgcagc 2280

agggccacag tgttgcgcat gggcaggtcc tcagctcgca ggtcccggca gcagagccgc 2340

agcgggctgc ccacgctgct tcggagtgcc tcccgcagga acgcatagga ggcccggttc 2400

acccgcaggt ccacgcgcac ctccacgggg atgggggctg gcccaggctc tgcggcccca 2460

ccctgctgct gggcaatgca tgtgcgagct acggcagcag tacagtccca catgctcatg 2520

gtgccaggat cctgttccac accatcatcc aagaggcccg tcatgtccag cacccgcagc 2580

gcatgcttcc tgggagtagg aatggtgtga ggaaggggtg gaggggcagc gcccataccc 2640

cctcccagcc accttgggac aggagcaacg cgcctgctcc tgcatgagct cttgctaact 2700

ttatgaccca agcctcctca ggggacccac ttacacaccc ctggcctgac gaccctccag 2760

ataagtccat acctgcagag gggctgtgtg ctggccccag gctctgaggt gtggagccgg 2820

gcagtcagcc ccaggataac agcctgcatg ctctcagtgc taggccgctc ctgcaggagg 2880

gcacggctgc agtgggcaca ctcctgtagc agctgctgga aactgagcag cgggaagggc 2940

cacgtgtgta ccaactcgcg cagtaccact gtcttcttgt ccatgaaggc caccttgaac 3000

agcaggggga agagttcgcg tggcagcaag ggcagggcct ggcaggcagc tggctggcac 3060

tgcagcacct gccgtgtgct caagaacaca agcgtgtgca tggtgctggg ccgggcagac 3120

ggccacctgc aggagaggga cttcagcagg gaggagagac cctaggccac taccaagcca 3180

cacacaagtg gtaggaaggc ccagcatagt gcctgaaaca aagtcagcag ttagcagtgg 3240

ccatggcaca gagaagcacc tattagatgc ctagcgctgg gctgggtgca gtgaactggc 3300

agacaaactc acaactcagc cccagccctc cagatgcaca ggatctcttt agagaaacta 3360

agcgtcaagc agtgtggcat cgtcaacaac tcgcttgctt gggtatgctt ttgacagatg 3420

cctgttgcct tgcagggccc cccatgggta cttcaggcct ctcaaattac aaagctgctt 3480

cacaaaacct tcttcgaaac ctgaataaag cccaggactc tccccaccaa ccttcatcag 3540

cattagtatt gacgggacac tctaaggagt caagcagcca aatcaccttt ccctcccctc 3600

tcctcacaga tgctctccag ggctacactt ggactccaca aaacaccaaa gagctggaag 3660

cacagaagca gcttcataag caacaaggca ggttgaaaag accgagaccc agggaacacc 3720

gagtccagat gatgtagcct ataaaataca agcccccgca aaagctacta ttctccagag 3780

cgtcaccaag cacggagacc ccgaaagaaa gcaaccctca cctttgttgg cccttaccac 3840

tggcctaaac ctctccctct tccctggaga gccactgtgg aaagaccaag tcttaagtat 3900

gtgggattca gaaggtgcaa agctacagac tgctcagaca aggcaaagaa agctgcctca 3960

gcagcaccca agctgcagag atgcacaaac actcccaagg ccacaaggta cctgtgacac 4020

agggtgccag ccctgctgct gcctgtagca tcctcaaaac taaatcgcag ccatctccct 4080

gtgacagatt agaaaatgaa ggcctcggcc tggcccaagg tcacagcctg agtgacaagc 4140

cagggaaggg ggccggggaa gacactctgg ccccaaggcc cttctttttg tcgacttttc 4200

cacatacaag taagtcctta aggcaccggt tcctgaaaag tcccagacac caaccctcgg 4260

cctagttctg ccggtggaca gatctcaacg atcatactcg ctctgacagg acagaccaac 4320

cgagcacttg tcacgggaga acaccaaagc agacggcctg cccaccaagg gaggcaggca 4380

cctccgtgcg acgccccctc ccctcccgcc ggccgcaggg aacgcgacgg tcctcggtcg 4440

cctgcgtttc gcgaagacgc ccccgcccgg ctcctccggg cctcgagccg cgggaggcgc 4500

tggaccctcc gctctttcgc ctcccgagcg gggcctgctc ctccaggtcg gatgcgtctc 4560

ccaccagggc ctgacgccgc tccgaccggc ccggggactc ccagtccttc ccggcccgcg 4620

gtggcacctc ccaggctccc ggcctcggcc ccgggctccc aaatgcagcc actgcctccc 4680

tcggccaggc cgccccgagc gaccggtgcc ccgccccttg aggccaggca gggccagggg 4740

cgtgcgccgc cccgctcaga cacccccccg gccgcccgcg ctcaccggtc ccgcaaccgc 4800

agccaccgcc tccagccccg cctagaccgt ccgccgctcc ccgcccggcg ccgcggcgcc 4860

ccgcgatgac gttgacgcct cccattggct gcttgtccga ggcccgacgg actggctgcc 4920

caggggcggt ggccccgccc ccggcccgcc gcgcatccgc attggctgtc ggcccccgcg 4980

acggctgcgc gggagattcg ctggacgatc gcaagcgcgg aggccgggcg ggcgcgcgcg 5040

ccatggagcg gctgcgggac gtgcgggagc ggctgcaggc gtgggagcgc gcgttccgac 5100

ggcagcgcgg gcggcgaccg agccaggtgc gggctgccca ggggccgagg ggctgagggc 5160

gcggcccgcg gctgacgcgt tccctttaca ggacgacgtg gaggcggcgc cggaggagac 5220

ccgcggtgag cgcgcggcgg ggcggcgggg gcgagaagac accgggtcgg caggggccca 5280

ggccccaccc tgaccccgcc tcccgctcgc ccacgcagcg ctctaccggg aataccgcac 5340

tctgaagcgt accacgggcc aggccggcgg cgggctccgc agctccgagt cgctccccgc 5400

ggcggccgaa gaggtaccca ggccccgccg ccccagcctc ctcccacttc cctgtttggc 5460

ggagtggcgg gagccacgga gtcgcggcca ggccgccgtg gggcacagaa cttgggaggg 5520

ggactgggca aagtgaagaa gggccgggcc tcgctccagg tgcgggaggg gtggctggga 5580

gcgcttctgc cgccacaaca gccttttctg gcctgtgccc ctgttgtctc ctgcaggcgc 5640

cagagccccg ctgctggggg ccccatctga atcgggctgc gaccaagagt ccacagccta 5700

cgccagggcg gagccgccag ggctcggtgc cggactacgg gcagcggctc aaggccaatc 5760

tgaaaggcac cctgcaggtg aggagtgggc aggcagtgag tccacgctag gtccacagct 5820

gcttccggtc cgggtcgccc tcttgtcatt ttttccacac agacaggcac gggcccctgt 5880

gccaaccagg gcacgagtct tcagggagct tctcggggcc ttcgcccttg actccctttc 5940

tagtccagcc ttgtgctaat tagcctgctc tacaattgag cgtggggact caggtaggtt 6000

ttagagtcta cagtagctca ggggcctgag ttcctcctgc tgttctgttg ttcccctccc 6060

aggccggacc agccctgggc cgcagaccgt ggcctctagg aagagcctca tctaaggcat 6120

ccaccccaaa gcccccaggt acagggcctg tcccctcctt tgcagaaaaa gtcagtgatg 6180

agcctccaca gctccctgag ccccagccaa ggccaggccg gctccagcat ctgcaggcat 6240

ccctgagcca gcggctgggc tccctagatc ctggctggtt acagcgatgt cacagtgagg 6300

tcccagattt tctgggggcc cccaaagcct gcaggcctga tctaggctca gaggaatcac 6360

aacttctgat ccctggtgag tcggctgtcc ttggtcctgg tgctggctcc cagggcccag 6420

aggcttcagc cttccaagaa gtcagcatcc gtgtggggag cccccagccc agcagcagtg 6480

gaggcgagaa gcggagatgg aacgaggagc cctgggagag ccccgcacag gtccagcagg 6540

agagcagcca agctggaccc ccatcggagg gggctggggc tgtagcagtt gaggaagacc 6600

ctccagggga acctgtacag gcacagccac ctcagccctg cagcagccca tcgaacccca 6660

ggtaccacgg actcagcccc tccagtcaag ctagggctgg gaaggctgag ggcacagccc 6720

ccctgcacat cttccctcgg ctggcccgcc atgacagggg caattacgta cggctcaaca 6780

tgaagcagaa acactacgtg cggggccggg cactccgtag caggctcctc cgcaagcagg 6840

taagacagcg acgggccagg acaggcattc cctttccctc ccctcagccc tcccgtattt 6900

cccgcccagt gaccctccta tgtgggcacc ccccaggcat ggaagcagaa gtggcggaag 6960

aaaggggagt gttttggggg tggtggtgcc acagtcacaa ccaaggagtc ttgtttcctg 7020

aacgagcagt tcgatcactg ggcagcccag tgtccccggc caggtgagac atctgccctg 7080

gagggtgggt ccggccaaca ctgtggagag ggcgcagtgc tcttttgggg gacacttatg 7140

ttccaagcaa caggccttcc aggtacccct ggtccaggcc ctaccctagc tcccctgaag 7200

gagggtggca gggacgacga tggctgtcac tcttttctgc tttggaaaaa gtagcccaga 7260

ggaagggcac tgcctgctgc caaccccctt tgggggaagg agaggttgtg gccagtggtt 7320

gtcttgcccg acctggagct cccattctac cctctcctgc ctgccccagc aagtgaggaa 7380

gacacagatg ctgttgggcc tgagccactg gttccttcac cacaacctgt acctgaggtg 7440

cccagcctgg accccaccgt gctgccactc tactccctgg ggccctcagg gcagttggca 7500

ggtgagcagt cagcttctgg cccagagcct tcactgaggg gttggggtga ctcaagtcat 7560

ggtgatcaac atctgtgtct gcagagacgc cggctgaggt gttccaggcc ctggagcagc 7620

tggggcacca agcctttcgc cctgggcagg agcgtgcagt catgcggatc ctgtctggtg 7680

agcgtggctg ccagggctga ggctgggctg aggccaggct gcagaaccct gctgctgact 7740

cccgccccat ccaggcatct ccacgctgct ggtgctgcct acaggtgccg gcaagtccct 7800

gtgctaccag ctcccagcgc tgctctacag ccggcgcagc ccctgcctca cgttggtcgt 7860

ctctcccctg ctgtcactca tggatgacca ggtgtgcaca cagggccctg ggcacacgta 7920

cacagccaag aaccagcact tgtgactccc aagggcaact gctgcttgtc ccctaaccac 7980

cccctcccct gggagcttca aggtgtctgt ggcctcagtc ccagtcttgg cagcaggtca 8040

aaggcagccc agctccacag gcaccacagc cacccctacg ggaaatgtgc tgggaaagga 8100

gccatcccta cttcagtctg tctgctctgg ggctcctggg ccaaggccca caggtggctc 8160

taaaccctta gccctaggac ccaggacctg gttctcctct cccctgaggg actaggatgg 8220

acatggcagc agctctggga tgacttgggg aagggccagg gctgggctgg cgtatgacgg 8280

ctgtcgctcc tgcatttgca ggtgtctggc ctgccaccgt gtctcaaggc ggcctgcata 8340

cactcgggca tgaccaggaa gcaacgggaa tctgtcctgc agaaggtggg ggcctcatgg 8400

gcctaggggt gagggaggca gcgggcgggc acctgggctg tgcctctgat cttgctgcct 8460

tcagattcgg gcagcccagg tacacgtgct gatgctgaca cctgaggcac tggtgggggc 8520

gggaggcctc cctccagccg cacagctgcc tccagttgct tttgcctgca ttgatgaggc 8580

ccactgcctc tcccagtggt cccacaactt ccggccctgc tacctgcgcg tctgcaaggt 8640

gagccatatg tgaactgggg tgggcggcca gggccgggat gggctggcgg gcctcacacc 8700

actgccgcct ctggtgcagg tgcttcggga gcgcatgggc gtgcactgct tcctgggcct 8760

cacagccaca gccacacgcc gcactgccag tgacgtggca cagcacctgg ctgtggctga 8820

agagcctgac ctccacgggc cagccccagt tcccaccaac ctgcaccttt ccgtgtccat 8880

ggacagggac acagaccagg tgggtgtgtg tgctctgggg accctgcagg gccctggctg 8940

ctgactgccc acgccgaccc ctcctcactc cccaccgccc acgccgaccc cttctcactc 9000

cccaccgccc acgccgaccc ctcctcactc cccacgccga cccctcctca ctccccactg 9060

cacacgccga cccctcctca ctccccactg cccacgccaa ccgctcctca tcaggcactg 9120

ttgacgctgc tgcaaggcaa acgttttcaa aacctcgatt ccattatcat ttactgcaac 9180

cggcgcgagg acacagagcg gatcgctgcg ctcctccgaa cctgcctgca cgcagcctgg 9240

gtcccagggt ctggaggtgc ggcatggaca gagctggtgt ccccgtggac ccaccttggg 9300

cacacatggt cccatcccac tgaccatctg cctgtcttcc ccaaaggtcg tgcccccaaa 9360

accacagccg aggcctacca cgcgggcatg tgcagccggg aacggcggcg ggtacagcga 9420

gccttcatgc agggccagtt gcaggtggtg gtggccacgg tggcctttgg gatggggctg 9480

gaccggccag atgtgcgggc tgtgctgcat ctggggctgc ccccaagctt cgagagctac 9540

gtgcaggccg tgggccgggc cgggcgtgac gggcagcctg cccactgcca cctcttcctg 9600

cagccccagg ttggcacccc ccccccccac actgccagtg ctcgagcccc cagtggtcca 9660

ccccaccctc atgaaagttg ccctgcaggg cgaagacctg cgagagctgc gcagacatgt 9720

gcacgccgac agcacggact tcctggctgt gaagaggctg gtacagcgcg tgttcccagc 9780

ctgcacctgc acctgcacca ggccgccctc ggagcaggaa ggggccgtgg gtggggagag 9840

gcctgtgccc aagtaccccc ctcaagaggc tgagcagctt agccaccaag cagccccagg 9900

acccagaagg gtctgcatgg gccatgagcg ggcactccca atacagctta ccgtacaggc 9960

tttggacatg ccggaggagg gtgaggaacc tggggtaagc cacaggggtg tggaggggct 10020

gtccccgcgt ccgctgagcc ctgctctgcc cccagccatc gagactttgc tgtgctacct 10080

ggagctgcac ccacaccact ggctggagct gctggcgacc acctataccc attgccgtct 10140
gaactgccct gggggccctg cccagctcca ggccctggcc cacaggtaag cacgccctgc 10200
ccagttggag acgaggttgg agaatcaggg ctgttggcca catgtccctt tttccctggg 10260
cacaggtgtc cccctttggc tgtgtgcttg gcccagcagc tgcctgagga cccagggcaa 10320
ggcagcagct ccgtggagtt tgacatggtc aagctggtgg actccatggg ctgggagctg 10380
gcctctgtgc ggcgggctct ctgccagctg cagtgggacc acgagcccag gacaggtgcg 10440
cctctcccca ccccacaccg ccctggacgc tgcctgcctg catctgacat gctttccggc 10500
aggtgtgcgg cgtgggacag gggtgcttgt ggagttcagt gagctggcct tccaccttcg 10560
cagcccgggg gacctgaccg ctgaggagaa ggaccagata tgtgacttcc tctatggccg 10620
tgtgcaggcc cgggagcgcc aggccctggc ccgtctgcgc agaaccttcc aggcctttca 10680
caggttggga ggaggcgggc ggggcctggg accatccacc ctcccgcagt gatcagctct 10740
gacaggctcc tccccacagc gtagccttcc ccagctgcgg gccctgcctg gagcagcagg 10800
atgaggagcg cagcaccagg ctcaaggacc tgctcggccg ctactttgag gaagaggaag 10860
ggcaggagcc gggaggcatg gaggacgcac agggccccga gccagggcag gccagagtga 10920
gtgcagtaag gccaggcagc tcatcggggt tgcaggttcc ctgggctgca tggggcttgc 10980
tctgtggatg cagtgccacg ggagctcaga ggaagcctga tgtgcctgtc cacacagctc 11040
caggattggg aggaccaggt ccgctgcgac atccgccagt tcctgtccct gaggccagag 11100
gagaagttct ccagcagggc tgtggcccgc atcttccacg gcatcggtga ggcctgggag 11160
gccccacccg ctgcaggctg gggctggggg ctggggcagg tgaggcctgg gaggctccac 11220
ccgctgcagg ctggggctgg ggctcacggc tgtgtcttgg ctccaccgta ggaagcccct 11280
gctacccggc ccaggtgtac gggcaggacc gacgcttctg gagaaaatac ctgcacctga 11340
gcttccatgc cctggtgggc ctggccacgg aagagctcct gcaggtggcc cgctgactgc 11400
actgcattgg gggatgtcgg gtagagctgg ggttgtcaga ggctagggca gtgactgagg 11460
acctgggcaa aacctgccac agggtgtggg aacgaggagg ctccaaaatg cagaataaaa 11520
aatgctcact ttgtttttat gggccctctt ggccaatgag gcaaccccca caagctgggg 11580
acaagcacat cacatattcc aggcacagag acatctgggc cacaggcaca tgtggcttta 11640
ttgaccactc cagccactca gctcagagaa aggtgctggg tgctaggtcc aggtacagaa 11700
cgggaaaggc agagaggatg agcaggagca agaagcaggg atgtggcccc aacccatcag 11760
ccaggcctcc ggccagagtg cccagcagca gcttccccag cagctccagc gtggccagaa 11820
ggctgtagtg tgtggcctga ggggggtggg ggtcacggtc aaggtcaggc ccacacagcc 11880
ccagccccgt ggtctctctt gtgtccctgc tacatctact cacctgcagg gccctggggg 11940

ccagctggct gcagcgcatc atcccagtga aggtgactgt ggtgaccagg cctcccaaga 12000

agtgctgcag acataggctc agcaaggctg accctgggag ggcagaagtc agcagtgccc 12060

caggctcctg gcccctctct tcctcctcaa ggccagcccc tcacctctca agattgtgcc 12120

agcgtccatg ctggccccca gggtgtccag gtggaagacc aaggcagtct gacaggctag 12180

gcccccgagg cggaagcgca gcaccgacct caacagaggc agcagtttcc tgggatgggg 12240

gtgggaaatg ggtcagtata gtggattggg ggcagctcag gtacaagtgg gtgggcaggg 12300

tacactgggg agggctcacc agtgcttggc cagcaaggtc ccacccaggg aggagccagc 12360

gatggagcag accacagcac ccacaccatt ccacagtccc aactcgggag cagaaacgcc 12420

gtggtccagc aggagaagag gaaacaggct gctggcaccc tgctcacctt gggtggtggg 12480

gggggcatga gcacgggagg ttccatatct gcccccacct cccttctccc cacctgggcc 12540

ctcagtcctg ccctgctctc ctcagtgttc cagagctgca gatactgggg gagaagtggg 12600

gcagcccgag agagcgctca gggatggggc tagagctggg ggcggacaag acacaaggcc 12660

cagctgcaga gccccggatg tcagggccaa gtcttgcagg tcatacccca gaaagagcgt 12720

ggccctgtaa tgggttgcaa gcccctgggg acacacctgg agctgtcccg gaagccccaa 12780

ctgctgttgc ctggctcgag gcctcactca cccagcttgt aggtgagcac aaagcctgcc 12840

gtccacacgg tccccggcac ggctagcacg tcccgcagaa ggtgcgcggt gtggggacgc 12900

tgctcggaag ggggctgctg tgggagccgc cgcagggctg gtgcagccca ggccagggcc 12960

gcggccagcc agtaggtggc agccaggagc agaaagagtt gcggccacga gaaggtgggc 13020

agcagcgcca gcagcgcgcc cccagctagc gcggccccca gcttgtacgc gaccacctgc 13080

acggtattgc ccggccccag ttcggccggc tccagcagct gcacagccag cgcgtccagg 13140

gccacatcct gcatggcggc acccaggttc aacaacagca gcaaccccgc cacagcggcg 13200

ggcagcccgg cctggccagc tccaggaggg ggcagcccgg caagcagccc acacaccagg 13260

cccaggcccg ccgtgctgcg cgtcacccag gccctcgccg agccctgcgc gtccaccagc 13320

ggggcccaag ccagcttgag cagccacgga gcgtacagaa ccttggccag ccccacgcgc 13380

gtcagcgaga ggccgccggc acgcagcagc actggcagga ggccggactg gagcccgtag 13440

ggcaggccct gcaccaggta gaggccggcc agcggcagca acttcccgcg catggcggct 13500

caggccggct gcgaggctgg gaggtcgggt tccaggtggg gtc 13543

<210> 7
<211> 20
<212> DNA
<213> Artificial Sequence

<220>
<223> Forward qPCR primer

<400>  7
gaggaggcca tcgagacttt                                                   20


<210>  8
<211>  20
<212>  DNA
<213>  Artificial Sequence

<220>
<223>  Reverse qPCR primer

<400>  8
gtataggtgg tcgccagcag                                                   20


<210>  9
<211>  20
<212>  DNA
<213>  Artificial Sequence

<220>
<223>  Forward Primer 1

<400>  9
caagtccctg tgctaccagc                                                   20


<210>  10
<211>  20
<212>  DNA
<213>  Artificial Sequence

<220>
<223>  Reverse Primer 1

<400>  10
cacaagtgct ggttcttggc                                                   20


<210>  11
<211>  19
<212>  DNA
<213>  Artificial Sequence

<220>
<223>  Forward Primer 2

<400>  11
tgaccatctg cctgtcttc                                                    19


<210>  12
<211>  17
<212>  DNA
<213>  Artificial Sequence

<220>
<223>  Reverse Primer 2

<400>  12
ccagccccat cccaaag                                                      17

<210>  13
<211>  20
<212>  DNA
<213>  Artificial Sequence

<220>
<223>  gRNA used for RECQL4 knockout

<400>  13
cgcactctga agctgaccac                                                    20